# FASCICULE DE BREVET EUROPEEN

(11) **EP 0 991 650 B1**
(45) Date de publication et mention de la délivrance du brevet: **10.09.2003**
(21) Numéro de dépôt: 98930827.5
(22) Date de dépôt: 11.06.1998
(51) Int. Cl.: C07D 471/04, A61K 31/40, C07D 403/12, A61K 31/495

(54) **NOUVEAUX COMPOSES DE N-BENZENESULFONYL-L-PROLINE, PROCEDE DE PREPARATION ET UTILISATION EN THERAPEUTIQUE**
N-BENZENESULFONYL-L-PROLIN-DERIVATE, VERFAHREN ZU IHRER HERSTELLUNG UND IHRE THERAPEUTISCHE VERWENDUNG
NOVEL N-BENZENESULPHONYL-L-PROLINE COMPOUNDS, PREPARATION METHOD AND USE IN THERAPY

(30) Priorité: 27.06.1997 FR 9708114
(43) Date de publication de la demande: 12.04.2000
(73) Titulaire: FOURNIER INDUSTRIE ET SANTE, F-75008 Paris (FR)
(72) Inventeur: DODEY, Pierre, F-21121 Fontaine-lès-Dijon (FR); BONDOUX, Michel, F-21121 Fontaine-lès-Dijon (FR); HOUZIAUX, Patrick, F-78580 Bazemont (FR); BARTH, Martine, F-78490 Monfort-l'Amaury (FR); OU, Khan, F-21121 Hauteville-lès-Dijon (FR)
(74) Mandataire: Hubert, Philippe
(86) Numéro de dépôt international: FR9801211
(87) Numéro de publication internationale: WO99000387

(56) Documents cités:
- EP-A- 0 622 361
- WO-A-96/33171
- WO-A-97/25315
- FR-A- 2 735 128
- FR-A- 2 737 892
- US-A- 4 217 130

## Description

### Domaine de l'invention

La présente invention concerne de nouveaux composés dérivés de la N-(benzènesulfonyl)-(L)-proline, leur procédé de préparation et leur utilisation en thérapeutique.

Ces nouveaux composés présentent une action antagoniste vis-à-vis de la bradykinine et sont utiles en thérapeutique, particulièrement pour le traitement de la douleur et de l'inflammation, et notamment dans le traitement de l'asthme, du choc traumatique cérébral et des rhinites allergiques.

### Art antérieur

On sait que l'une des possibilités de traitement de certaines pathologies à caractère douloureux et/ou inflammatoire (telles que l'asthme, la rhinite, le choc septique, la douleur dentaire, etc.) est d'inhiber l'action de certaines hormones telles que la bradykinine ou la kallidine. En effet ces hormones peptidiques sont impliquées dans un grand nombre de processus physiologiques dont certains sont liés de façon étroite à ces pathologies.

Bien qu'actuellement aucun produit possédant ce mode d'action ne soit encore commercialisé, de nombreuses études ont été entreprises pour comprendre le mode d'action des kinines et en particulier la bradykinine et ses homologues, puis pour créer des composés susceptibles d'être antagonistes des récepteurs de la bradykinine. Parmi les nombreuses publications relatant ces travaux, on peut citer Pharmacological Reviews Vol. 44 n° 1, pages 1-80 (1992) et Biopolymers (Peptide Science) vol. 37 pages 143-155 (1995).

La bradykinine est une hormone peptidique constituée de 9 aminoacides (Arg-Pro-Pro-Gly-Phe-Ser-Pro-Phe-Arg) et la kallidine est une hormone peptidique (Lys-Arg-Pro-Pro-Gly-Phe-Ser-Pro-Phe-Arg) qui comporte un aminoacide supplémentaire (Lys) par rapport à la bradykinine. On sait que des études antérieures ont permis d'obtenir des peptides qui interagissent avec les récepteurs de la bradykinine : certains comme le Bradycor (CP.0127 de la société Cortech), l'Icatibant (HOE 140 de la société Hoechst) ["Bradycor" et "Icatibant" sont des dénominations communes internationales (DCI)] ou encore le NPC 17761 (de la société Scios-Nova) présentent une action inhibitrice de la fixation de la bradykinine sur son récepteur B₂. Des publications récentes font état d'autres peptides susceptibles d'avoir une action antagoniste de la bradykinine vis-à-vis de son récepteur B₂ ; parmi celles-ci on peut citer par exemple WO-A-97/09347, WO-A-97/09346, US-A-5610140, US-A-5620958, US-A-5610142 et US-A-5597803. Par ailleurs, des composés non peptidiques ont été proposés comme antagonistes vis-à-vis de la fixation de la bradykinine sur son récepteur B₂, notamment dans EP-A-0596406, EP-A-0622361, US-A-5578601, FR-A-2735128, JP-A-09 040662, FR-A-2737892 et WO-A-97/11069. On sait en outre que certains composés de structure plus ou moins apparentée à celles des composés visés dans la présente demande ont déjà été décrits, notamment dans les publications DE-A-3617183 et dans EP-A-0261539, eu égard à leurs éventuelles propriétés antithrombotiques.

### But de l'invention

Il existe un besoin d'atténuer ou de supprimer chez les mammifères et surtout chez l'homme les douleurs et les inflammations.

Pour satisfaire ce besoin, on a recherché une nouvelle solution technique qui soit efficace dans le traitement des algies quelle que soit leur origine, notamment dans le traitement des algies liées à des phénomènes inflammatoires ou à des traumatismes.

Selon l'invention, on se propose de fournir une nouvelle solution technique, qui met en oeuvre une fixation compétitive au niveau du récepteur B₂ de la bradykinine entre (i) la bradykinine et les hormones apparentées ou analogues, et (ii) une substance antagoniste, et qui fait appel à des composés de type benzène-sulfonamide, structurellement différents des produits connus précités, et capables de limiter ou d'inhiber substantiellement la fixation de la bradykinine et des hormones analogues sur ledit récepteur B₂ de la bradykinine.

Suivant cette solution technique, les nouveaux composés se fixent de façon compétitive sur le récepteur B₂ de la bradykinine sans provoquer les effets de la bradykinine sur ce récepteur (ces nouveaux composés sont des substances dites antagonistes). Il s'en suit l'apparition d'un état analogue à celui observé en l'absence de bradykinine, à savoir une diminution de la douleur et des réactions inflammatoires.

Conformément à cette nouvelle solution technique on se propose de fournir, selon un premier aspect de l'invention, des composés dérivés de N-(benzènesulfonyl)-(L)-proline en tant que produits industriels nouveaux ; selon un second aspect de l'invention, un procédé de préparation de ces composés; et selon un troisième aspect de l'invention, une utilisation de ces composés notamment en thérapeutique en tant qu'agents antalgiques et/ou anti-inflammatoires.

### Objet de l'invention

Selon la nouvelle solution technique de l'invention, on préconise en tant que produit industriel nouveau, un composé de N-(benzènesulfonyl)-L-proline qui est caractérisé en ce qu'il est choisi parmi l'ensemble constitué par :
(i) les composés de formule I : 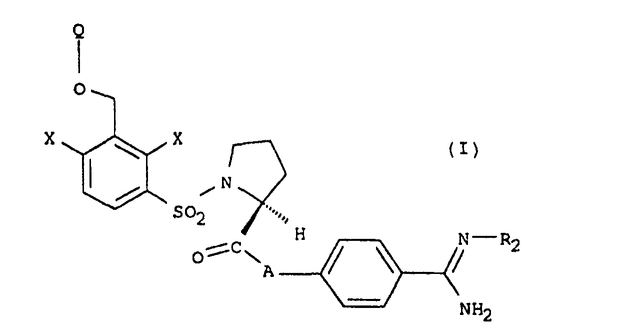 dans laquelle :
   X représente un atome d'halogène,
   A représente un groupe divalent : 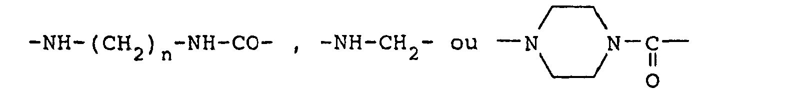
   Q représente un groupe : 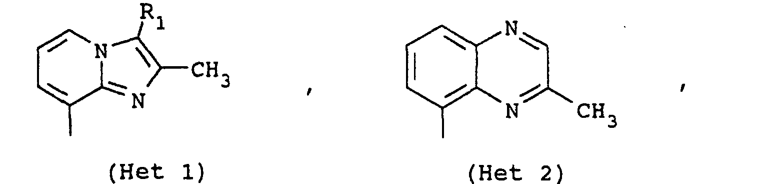 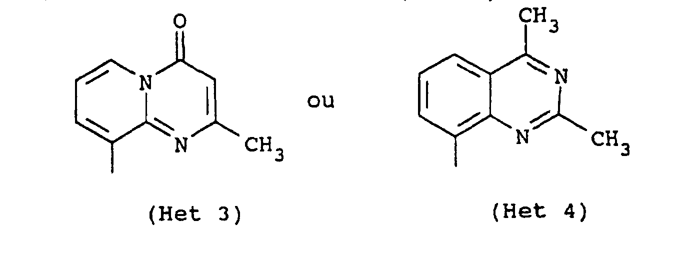
   R₁ représente un atome d'hydrogène, un atome d'halogène, un groupe alkyle en C₁-C₃ à chaîne hydrocarbonée linéaire ou ramifiée ou un groupe 1-oxoalkyle en C₁-C₅,
   R₂ représente un atome d'hydrogène ou un groupe OH,
   n représente 2, 3, ou 4, et
(ii) leurs sels d'addition.

Selon l'invention, on préconise aussi un procédé de préparation des composés de formule I et de leurs sels d'addition.

On préconise également l'utilisation d'une substance antagoniste d'un récepteur de la bradykinine et des hormones analogues, ladite utilisation étant caractérisée en ce que l'on fait appel à une substance antagoniste du récepteur B₂ de la bradykinine et choisie parmi les composés de formule I et leurs sels d'addition non-toxiques, pour l'obtention d'un médicament destiné à une utilisation en thérapeutique vis-à-vis des pathologies impliquant la bradykinine ou ses analogues, en particulier vis-à-vis des algies, et notamment dans le traitement ou la prévention de pathologies liées à des états inflammatoires ou douloureux.

### Description détaillée de l'invention

Dans la formule générale I des composés de l'invention, on entend par atome d'halogène un atome de fluor, de chlore, de brome ou d'iode, l'halogène préféré étant l'atome de chlore.

Par groupe alkyle en C₁-C₃ à chaîne hydrocarbonée linéaire ou ramifiée, on entend ici un groupe méthyle, éthyle, propyle ou 1-méthyléthyle.

Par groupe 1-oxoalkyle en C₁-C₅, on comprend les groupes acétyle, 1-oxopropyle, 1-oxobutyle et 2-méthyl-1-oxopropyle.

Dans le composé de formule I, l'hétérocycle azoté de structure pyrrolidine comprend 1 atome de carbone asymétrique. Selon l'invention, ce carbone est de configuration S, ce qui correspond à la configuration de la L-proline.

Par "sels d'addition", on entend les sels d'addition d'acide obtenus par réaction d'un composé de formule I avec un acide minéral ou un acide organique. Les acides minéraux préférés pour salifier un composé basique de formule I sont les acides chlorhydrique, bromhydrique, phosphorique et sulfurique. Les acides organiques préférés pour salifier un composé basique de formule I sont les acides méthanesulfonique, benzènesulfonique, maléique, fumarique, oxalique, citrique, lactique et trifluoroacétique.

Par "température ambiante" on entend ici une température de 15 à 25°C, et par "température voisine de la température ambiante" une température de 0 à 40°C et mieux de 10 à 35°C.

Le procédé général de préparation des composés de formule I, que l'on préconise selon l'invention, comprend selon une première variante A, les étapes consistant à :
(1) faire réagir un composé hétérocyclique hydroxylé de formule :

   Q-O-Met

   dans laquelle
   Met représente un métal alcalin, notamment Na ou K,
   Q représente un groupe hétérocyclique choisi parmi les structures Het 1, Het 2, Het 3 et Het 4 : 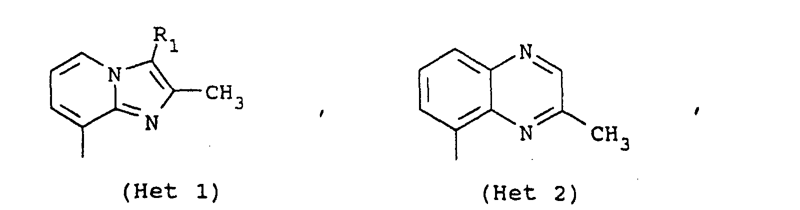 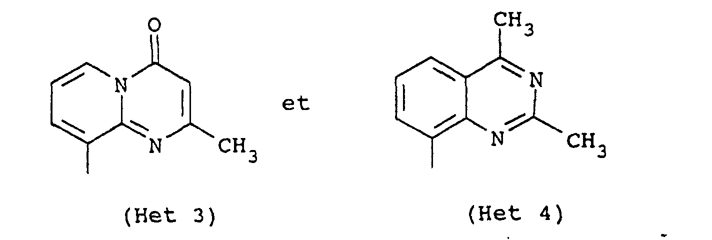
   R₁ représentant un atome d'hydrogène ou un groupe alkyle en C₁-C₃, avec un composé de formule II : 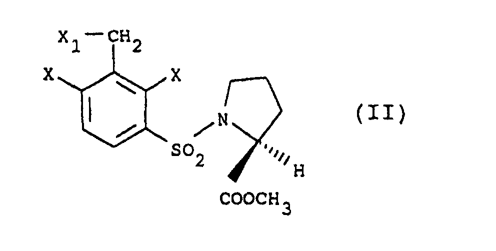
   dans laquelle X représente un atome d'halogène et X₁ représente un atome d'halogène, préférentiellement un atome de brome,
      dans un solvant anhydre comme par exemple le diméthylformamide, à une température comprise entre 0 et 50°C et pendant 0,5 à 10 heures, pour obtenir un composé de formule III : 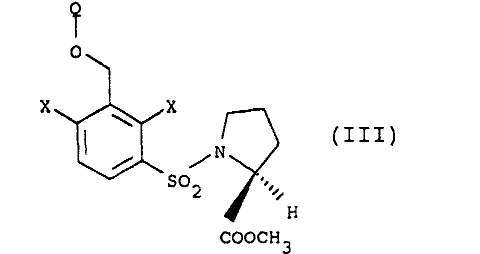 dans laquelle Q, X et R₁ conservent la même signification que précédemment ;
(2) si nécessaire, lorsque dans le composé de formule III Q représente le groupe Het 1 où R₁ est un atome d'hydrogène : 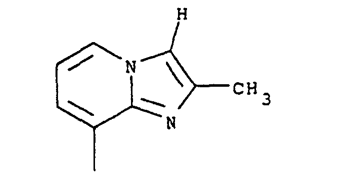 faire réagir ledit composé de formule III avec un agent halogénant tel que le N-bromo- ou le N-chlorosuccinimide, dans un solvant approprié, notamment un solvant halogéné, un éther ou un alcool, à une température comprise entre environ 0 et 50°C et pendant 0,5 à 20 heures, pour obtenir un composé de formule III' : 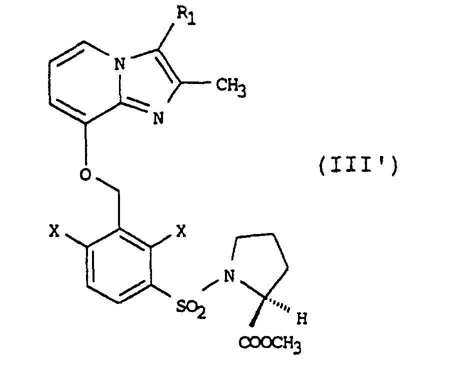 dans laquelle
   R₁ représente un atome d'halogène, préférentiellement le brome ou le chlore ;
(3) hydrolyser la fonction ester du composé de formule III ou III' obtenu selon l'une des étapes (1) ou (2) ci-dessus, notamment par réaction avec une solution aqueuse d'hydroxyde de sodium, dans un solvant miscible tel que du méthanol, à une température de l'ordre de 20 à 60°C et pendant 1 à 5 heures, pour obtenir après acidification un composé de formule IV : 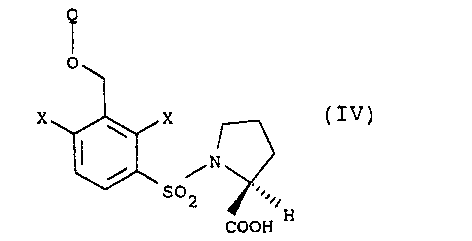 dans laquelle Q et X conservent la même signification que ci-dessus et R₁ représente un atome d'hydrogène, un atome d'halogène ou un groupe alkyle en C₁-C₃ ;
(4) faire réagir le composé de formule IV, ainsi obtenu, avec le sel d'une amine de formule : 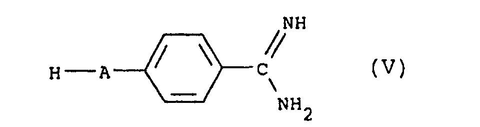 dans laquelle A représente un groupe 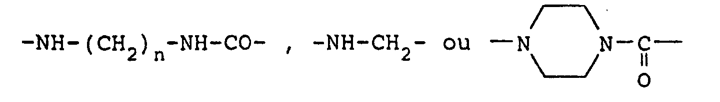 où n représente 2, 3 ou 4,
   dans un solvant approprié, notamment le dichlorométhane, en présence d'activateurs tels que notamment le 1-hydroxy-7-aza-benzotriazole (HOAT) et le chlorhydrate de 1-[3-(diméthylaminopropyl)-3-éthyl]carbodiimide (EDCI), à une température voisine de la température ambiante (10-35°C), pendant 2 à 50 heures, pour obtenir un composé de formule l où R₂ est H : 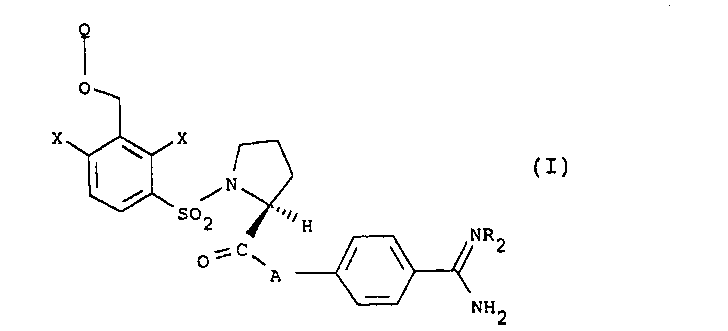 dans laquelle A, Q, X et R₁ conservent la même signification que précédemment et R₂ est H ; et,
(5) si nécessaire, faire réagir le composé de formule 1 ainsi obtenu, avec un acide pour obtenir le sel d'addition d'acide correspondant ; selon une seconde variante B, les étapes consistant à :
   (1) faire réagir le composé acide de formule IV obtenu , par exemple, à l'étape (3) de la variante A dans lequel R₁ est un atome d'hydrogène, un atome de chlore, un groupe alkyle en C₁-C₃ à chaîne hydrocarbonée linéaire ou ramifiée ou un groupe 1-oxoalkyle en C₁-C₅, avec un composé de formule VI : 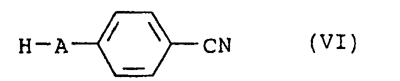 dans laquelle A représente un groupe 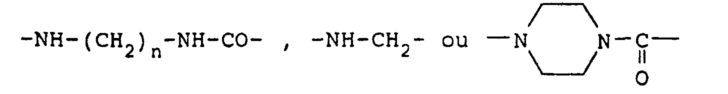 où n représente 2, 3 ou 4,
      dans des conditions analogues à celles préconisées pour réaliser l'étape (4) de la variante A ci-dessus, pour obtenir un composé de formule VII : 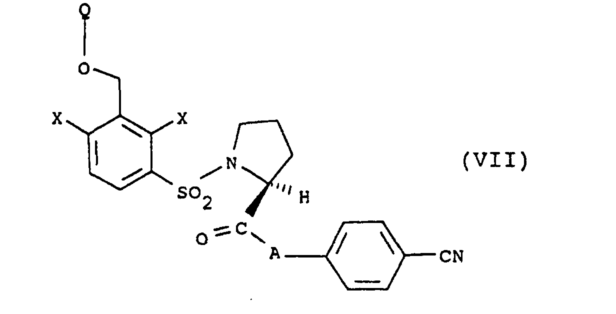 dans laquelle Q, R₁, X et A conservent la même signification que dans les produits de départ ;
   (2) faire réagir le composé de formule VII, ainsi obtenu, avec de l'hydroxylamine (libérée à partir de son chlorhydrate par action, dans le milieu réactionnel, d'une base forte aprotique telle que la triéthylamine), dans un solvant approprié, notamment aprotique tel que le diméthylsulfoxyde (DMSO), à température ambiante (15-25°C), pendant 1 à 12 heures, pour obtenir un composé de formule VIII : 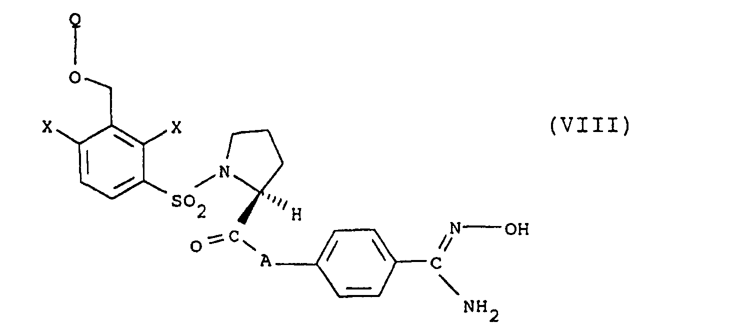 dans laquelle Q, R₁, X et A conservent la même signification que précédemment ;
   (3) acétyler le composé de formule VIII, ainsi obtenu, à une température voisine de la température ambiante pendant 1 à 8 heures, pour obtenir le composé de formule IX : 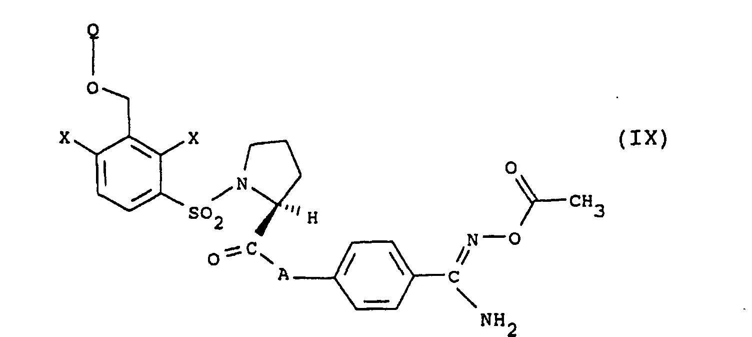 dans laquelle Q, R₁, X et A conservent la même signification que ci-dessus,
   (4) effectuer une réduction par hydrogénation catalytique du composé de formule IX, ainsi obtenu, notamment dans un solvant tel que par exemple le méthanol, en présence d'un catalyseur d'hydrogénation comme par exemple le catalyseur de Lindlar, à une température proche de la température ambiante, sous une pression d'hydrogène comprise entre 10⁵ et 10⁶ Pascals, pour obtenir le composé de formule I dans laquelle Q, R₁, X et A conservent la même signification que ci-dessus et R₂ est H ;
   selon une troisième variante C, les étapes consistant à :
   (1) faire réagir le composé de formule V obtenu conformément à l'étape (3) de la variante A avec une amine de formule 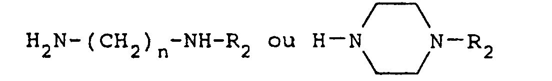 où n représente 2, 3 ou 4,
      R₂ représente un groupe aminoprotecteur comme par exemple le groupe " Boc " (1,1-diméthyléthoxycarbonyle), dans des conditions opératoires analogues à celles préconisées pour réaliser l'étape (4) de la variante A, pour obtenir un composé de formule X : 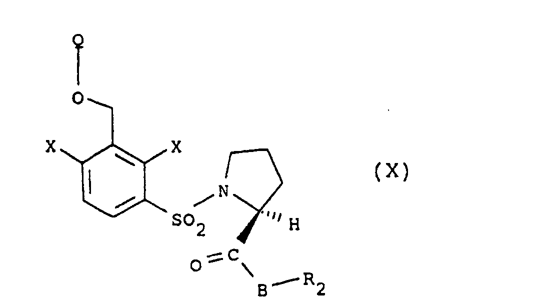 dans laquelle B représente 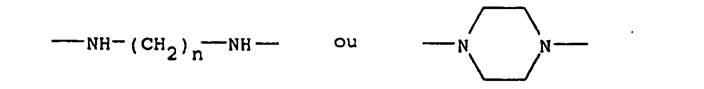 et Q, R₁ et X conservent la même signification que précédemment ;
   (2) effectuer la déprotection de la fonction amine du composé de formule X, ainsi obtenu, de façon à remplacer le groupe R₂ par un atome d'hydrogène, par exemple si R₂ est le groupe Boc par action d'un acide en solution, dans un solvant tel que par exemple de l'acétate d'éthyle, à température ambiante, pendant 4 à 30 heures, et ainsi obtenir le composé de formule XI : 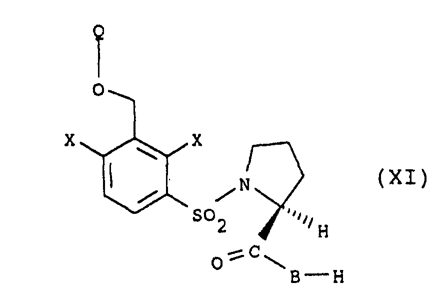 dans laquelle B, Q, R₁ et X conservent la même signification que ci-dessus ; (3) faire réagir le composé de formule XI, ainsi obtenu, avec l'acide 4-(aminoiminométhyl)benzoïque, dans des conditions analogues à celles décrites précédemment à l'étape (4) de la variante A ci-dessus, pour obtenir le composé de formule I où R₂ est H : 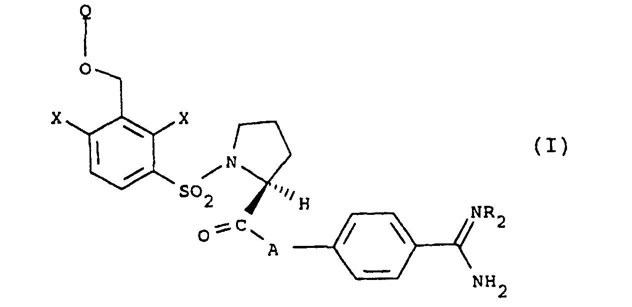 dans laquelle
      Q, R₁ et X conservent la même signification que précédemment, R₂ est H et
      A représente un groupe : 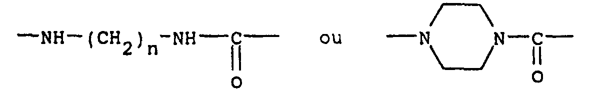
   et selon une quatrième variante D, les étapes consistant à :
   (1) faire réagir l'acide de formule : 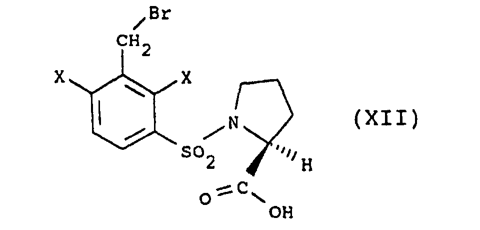 dans laquelle X représente un halogène,
      avec un composé de formule : 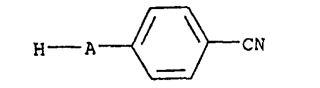 dans laquelle A représente un groupe : 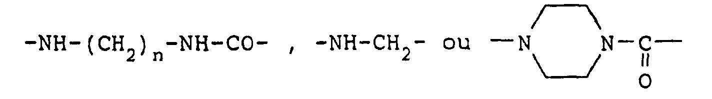 où n représente 2, 3 ou 4,
      dans des conditions réactionnelles analogues à celles préconisées pour réaliser l'étape (4) de la variante A ci-dessus, et obtenir le composé de formule XIII : 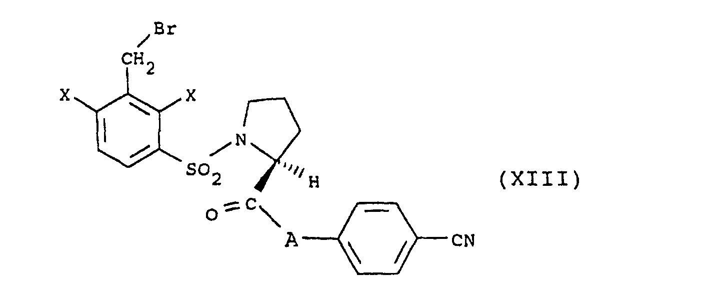 dans laquelle A et X conservent la même signification que ci-dessus ;
   (2) faire réagir le composé de formule XIII, ainsi obtenu, avec un dérivé hétérocyclique hydroxylé de structure générale Q-OH, Q étant choisi parmi les structures : 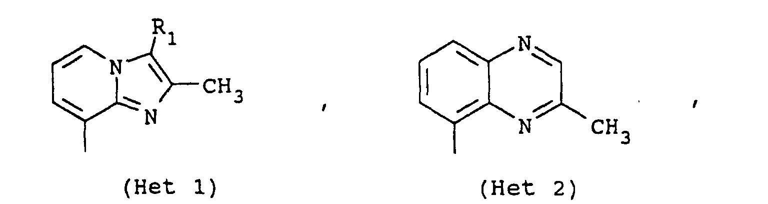 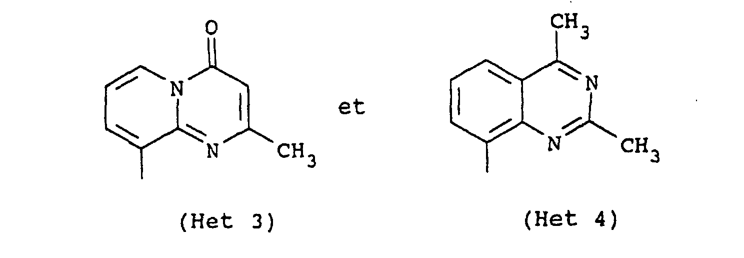 où R₁ représente un atome d'hydrogène, un atome d'halogène, un groupe alkyle en C₁-C₃, ou un groupe 1-oxoalkyle en C₁-C₅,
      dans des conditions opératoires analogues à celles décrites pour effectuer l'étape (1) de la variante A ci-dessus, pour obtenir le composé de formule XIV : 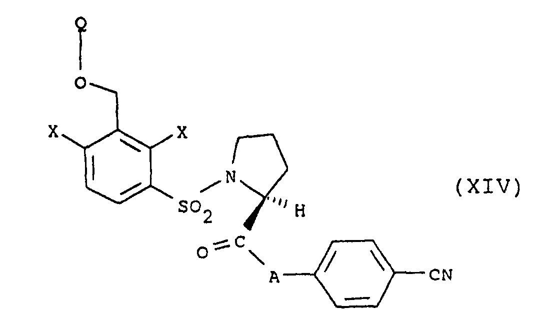 dans laquelle Q, R₁, X et A conservent la même signification que dans les produits de départ ; et,
   (3) effectuer ensuite sur le composé de formule XIV, ainsi obtenu, une série de réactions analogues à celles précédemment décrites aux étapes (2), (3) et (4) de la variante B ci-dessus, pour obtenir le composé de formule I selon l'invention dans laquelle Q, X, R₁ et A conservent la même signification que dans les composés de départ.

L'invention sera mieux comprise à la lecture qui va suivre (i) d'exemples de préparation et (ii) de résultats d'essais pharmacologiques réalisés avec des composés selon l'invention. Bien entendu l'ensemble de ces éléments n'est pas limitatif mais est donné à titre d'illustration.

Dans le cas de composés présentant dans leur structure un carbone asymétrique, l'absence d'indication particulière ou la mention (R,S) signifie qu'il s'agit de composés racémiques ; dans le cas de composés présentant une chiralité, celle-ci est indiquée à la suite immédiate de l'indexation du substituant porté par ledit carbone asymétrique ; on utilise alors les signes (R) ou (S), conformément aux règles de Cahn, Ingold et Prelog. La nomenclature utilisée dans les exemples est celle préconisée par les Chemical Abstracts : ainsi certains dérivés de la L-proline peuvent devenir, après réaction de la fonction acide avec une aminé, des dérivés de la 2(S)-pyrrolidinecarboxamide.

Dans la partie expérimentale, les "préparations" sont relatives aux composés intermédiaires et les "exemples" sont relatifs aux produits selon l'invention.

Les points de fusion (F) indiqués ci-après sont en général mesurés à l'aide d'un banc Koffler et ne sont pas corrigés, il s'agit alors de points de fusion instantanée.

Les caractéristiques spectrales des signaux de résonance magnétique nucléaire (RMN) sont données pour le proton (¹H) ou pour l'isotope 13 du carbone (¹³C) : on indique le déplacement chimique par rapport au signal du tétraméthyl-silane et, entre parenthèses, la forme du signal (s pour singulet, d pour doublet, t pour triplet, q pour quadruplet, m pour multiplet, sl pour signal large) et le nombre de protons concernés par le signal. A titre indicatif, les spectres RMN ¹H ont été réalisés à 300 MHz.

### PREPARATION I

### N-[[3-[[2-Méthylimidazo[1,2-a]pyridin-8-yl]oxyméthyl]-2,4-dichlorophényl]-sulfonyl]-L-proline, méthyl ester

On prépare une solution de 10 g (67.10⁻³ mole) de 2-méthyl-8-hydroxy-imidazo[1,2-a]pyridine dans 300 ml de diméthylformamide (DMF) et on ajoute 2,02 g (67.10⁻³ mole) d'une suspension d'hydrure de sodium à 80 % dans l'huile. On agite le mélange à température ambiante pendant 30 mn puis on ajoute une solution de 2,91 g (67.10⁻³ mole) d'ester méthylique de la N-[[3-(bromométhyl)-2,4-dichlorophényl]sulfonyl]-L-proline dans 100 ml de DMF. Après agitation à température ambiante pendant 15 heures, le mélange réactionnel est versé sur 500 ml d'eau et extrait 3 fois avec de l'acétate d'éthyle. Les phases organiques rassemblées sont lavées à l'eau, séchées sur sulfate de sodium et concentrées sous pression réduite. Le produit brut ainsi obtenu est purifié par chromatographie surgel de silice (gel de silice 60 de granulométrie 15 à 40 µm), avec pour éluant un mélange toluène/2-propanol (96/4 ; v/v). On obtient ainsi 22,5 g du produit attendu sous forme d'un solide légèrement rose (Rendement = 67 %).
F = 149°C
[α]²³_{D} = - 19,0° (c = 1,5 ; CHCl₃)

### PREPARATION II

### N-[[3-[[2-Méthylimidazo[1,2-a]pyridin-8-yl]oxyméthyl]-2,4-dichlorophényl]-sulfonyl]-L-proline

On prépare une solution de 12,2 g (24.10⁻³ mole) du composé obtenu selon la préparation I, dans 180 ml de méthanol et 120 ml d'eau, puis on ajoute 48 ml d'une solution 1N d'hydroxyde de sodium. On porte le mélange réactionnel à 40°C et on maintient sous agitation pendant 1,5 heure. On évapore le méthanol sous pression réduite, refroidit, ajoute 200 ml d'eau et acidifie sous agitation jusqu'à pH2 par addition lente d'une solution d'acide chlorhydrique 1N. On extrait le milieu avec du dichlorométhane et la phase organique obtenue est séchée sur sulfate de sodium et concentrée sous pression réduite. On obtient ainsi 11,6 g du produit attendu sous forme de cristaux blanc-rosé.
F = 135°C
[α]²²_{D} = - 50,7° (c = 1,02 ; CH₂Cl₂)

### PREPARATION III

### Acide [3-[(4-cyanobenzoyl)amino]propyl]carbamique, 1,1-diméthyléthyl ester

On prépare une solution de 10 g (57.10⁻³ mole) d'acide (3-amino-propyl)carbamique, 1,1-diméthyléthyl ester (ou N-Boc-1,3-propanediamine) dans 75 ml de dichlorométhane et on ajoute 15,9 ml (114.10⁻³ mole) de triéthylamine. On refroidit le mélange avec un bain de glace et ajoute progressivement 10,38 g (60.10⁻³ mole) du chlorure de l'acide 4-cyano-benzoïque. On laisse revenir le milieu réactionnel à température ambiante, le maintient sous agitation pendant 10 heures, puis le verse sur 150 ml d'eau. On extrait avec du dichlorométhane et la phase organique obtenue est lavée avec une solution d'acide chlorhydrique 1N, puis avec de l'eau et enfin séchée sur sulfate de sodium et concentrée sous pression réduite. On obtient ainsi 16,4 g du produit attendu sous forme de cristaux ocre (Rendement =
94 %).
RMN¹H (DMSO) : 1,37 (s,9H) ; 1,63 (m,2H) ; 2,97 (m,2H) ; 3,26 (m,2H) ; 6,84 (t,1H) ; 7,97 (s,4H) ; 8,70 (t, 1H).

### PREPARATION IV

### Chlorhydrate de N-(3-aminopropyl)-4-cyanobenzamide

On dissout 16,4 g (54.10⁻³ mole) du composé obtenu selon la préparation III dans 150 ml d'acétate d'éthyle, puis on ajoute 104 ml d'une solution de chlorure d'hydrogène à 2,6 mole/l dans l'acétate d'éthyle. Le mélange est agité à température ambiante pendant 24 heures, puis concentré sous pression réduite. Le produit brut, ainsi obtenu, est lavé avec de l'éther éthylique et séché sous vide à 40°C. On obtient ainsi 12,15 g du produit attendu sous forme d'un solide blanc-cassé (Rendement = 94 %).
F = 176-180°C

### PREPARATION V

### 1-[[3-[[2-Méthylimidazo[1,2-a]pyridin-8-yl]oxyméthyl]-2,4-dichlorophényl]-sulfonyl]-N-[3-[(4-cyanobenzoyl)amino]propyl]-2(S)-pyrrolidinecarboxamide

On prépare une solution de 5,8 g (12.10⁻³ mole) du composé obtenu selon la préparation II, dans 200 ml de dichlorométhane, puis on ajoute 2,53 g (13.10⁻³ mole) de EDCI [1-(3-diméthylaminopropyl)-3-éthyl-carbodiimide] et 1,79 g (13.10⁻³ mole) de HOAT (1-hydroxy-7-azabenzotriazole). Le mélange résultant est agité 30 mn à température ambiante puis on y ajoute une solution de 2,69 g (12.10⁻³ mole) du composé obtenu selon la préparation IV dans un mélange de 150 ml de dichlorométhane et de 2,6 ml (24.10⁻³ mole) de N-méthylmorpholine. Le milieu réactionnel est maintenu sous agitation pendant 16 heures à température ambiante, puis lavé successivement avec de l'eau, une solution d'acide chlorhydrique 0,5N, une solution de bicarbonate de sodium et enfin à nouveau avec de l'eau. Après déshydratation sur sulfate de sodium, la phase organique est concentrée sous pression réduite et le produit brut obtenu est purifié par chromatographie sur gel de silice avec pour éluant un mélange dichlorométhane/méthanol (97/3 ; v/v). On obtient ainsi 6,66 g du produit attendu sous forme de cristaux blancs (Rendement = 83 %).
F = 100-102°C
[α]²³_{D} = - 15° (c = 0,99 ; CH₂Cl₂)

### Exemple 1

### 1-[[3-[[2-Méthylimidazo[1,2-a]pyridin-8-yl]oxyméthyl]-2,4-dichlorophényl]-sulfonyl]-N-[3-[[4-[(amino)(hydroxyimino)méthyl]benzoyl]amino]propyl]-2(S)-pyrrolidinecarboxamide

On prépare une solution de 6,53 g (9,7.10⁻³ mole) du composé obtenu selon la préparation V dans 100 ml de diméthylsulfoxyde (DMSO) et on ajoute 1,36 g (19.10⁻³ mole) de chlorhydrate d'hydroxylamine, puis 2,7 ml (19.10⁻³ mole) de triéthylamine. On agite le mélange réactionnel pendant 3 heures à température ambiante, puis on ajoute à nouveau une même quantité de chlorhydrate d'hydroxylamine et de triéthylamine et on maintient sous agitation pendant 8 heures. Le milieu réactionnel est versé sur 400 ml d'eau sous agitation et le précipité obtenu est séparé par filtration et repris en solution dans du dichlorométhane. La phase organique obtenue est lavée à l'eau puis séchée sur sulfate de sodium. Après élimination du solvant sous pression réduite, on obtient 5,37 g du produit attendu sous forme de cristaux blancs (Rendement = 78 %).
F = 135°C
[α]²²_{D} = - 6,9° (c = 1,04 ; DMSO)

### PREPARATION VI

### 1-[[3-[[2-Méthylimidazo[1,2-a]pyridin-8-yl]oxyméthyl]-2,4-dichlorophényl]-sulfonyl]-N-[3-[[4-[(acétoxyimino)(amino)méthyl]benzoyl]amino]propyl]-2-(S)-pyrrolidinecarboxamide

On prépare une solution de 5,28 g (7,5.10⁻³ mole) du composé obtenu selon l'exemple 1 dans 150 ml de dichlorométhane et on ajoute 0,96 ml (10.10⁻³ mole) d'anhydride acétique. Après agitation à température ambiante pendant 15 heures, le milieu réactionnel est concentré sous pression réduite et on obtient ainsi 5,48 g du produit attendu sous forme de cristaux beiges.
F = 105-107°C
[α]²⁰_{D} = - 19° (c = 1,0 ; CH₃OH)

### Exemple 2

### 1-[[3-[[2-Méthylimidazo[1,2-a]pyridin-8-yl]oxyméthyl]-2,4-dichlorophényl]sulfonyl]-N-[3-[[4-(aminoiminométhyl)benzoyl]amino]propyl]-2(S)-pyrrolidinecarboxamide

On prépare une solution de 5,4 g (7,2.10⁻³ mole) du composé obtenu selon la préparation VI dans 150 ml de méthanol et on ajoute 1,08 g de catalyseur de Lindlar (à 5 % de palladium). Le mélange est agité sous atmosphère d'hydrogène, sous une pression de 3.10⁵ Pascals, pendant 5 heures à température ambiante. Après séparation du catalyseur par filtration, le solvant est éliminé par évaporation sous pression réduite et le produit brut est purifié par chromatographie sur gel de silice greffé NH₂ ("Lichroprep NH₂") avec pour éluant un mélange dichlorométhane/méthanol (95/5 ; v/v). On obtient ainsi 2,02 g du produit attendu sous forme de cristaux blancs (Rendement = 41 %).
F = 120-125°C
[α]²⁵_{D} = - 36° (c = 1,03; CH₃OH)

### Exemple 3

### 1-[[3-[[2-Méthylimidazo[1,2-a]pyridin-8-yl]oxyméthyl]-2,4-dichlorophényl]-sulfonyl]-N-[3-[[4-(aminoiminométhyl)benzoyl]amino]propyl]-2(S)-pyrrolidinecarboxamide, méthanesulfonate

On prépare une solution de 1,95 g (2,8.10⁻³ mole) du composé obtenu selon l'exemple 2 dans 14 ml de méthanol. On ajoute sous agitation 184 µl (2,8.10⁻ ³ mole) d'acide méthanesulfonique. Après 30 mn sous agitation à température ambiante, le milieu réactionnel est versé sur 500 ml d'éther éthylique. Le précipité obtenu est filtré, lavé à l'éther et dissous dans 60 ml d'eau distillée. La solution obtenue est lyophilisée et on obtient 2,07 g du produit attendu sous forme de cristaux blancs (Rendement = 94 %).
F = 170-172°C
[α]²⁵_{D} = - 38,2° (c = 1,03 ; CH₃OH)

### PREPARATION VII

### Acide 4-(4-cyanobenzoyl)-1-pipérazinecarboxylique, 1,1-diméthyléthyl ester

En opérant de façon analogue à la préparation III, au départ de l'acide 1-pipérazinecarboxylique, 1,1-diméthylethylester, on obtient le produit attendu sous forme de cristaux blancs (Rendement = 90 %).
RMN¹H : 1,46 (s,9H) ; 3,38 (m,4H) ; 3,51 (m,2H) ; 3,73 (m,2H) ; 7,50 (d,2H) ; 7,73 (d,2H).

### PREPARATION VIII

### Chlorhydrate de 1-(4-cyanobenzoyl)pipérazine

En opérant de façon analogue à la préparation IV, au départ du composé obtenu selon la préparation VII, on obtient le produit attendu sous forme de cristaux blancs (Rendement = 99 %).
F = 262-264°C

### PREPARATION IX

### 1-[[3-[[2-Méthylimidazo[1,2-a]pyridin-8-yl]oxyméthyl]-2,4-dichlorophényl]-sulfonyl]-2(S)-[[4-(4-cyanobenzoyl)pipérazin-1-yl]carbonyl]pyrrolidine

En opérant de façon analogue à la préparation V, au départ du composé obtenu selon la préparation VIII, on obtient le produit attendu sous forme de cristaux blancs (Rendement = 93 %).
F = 112-115°C
[α]²¹_{D} = + 2,8° (c = 1,03 ; CH₂Cl₂)

### Exemple 4

### 1-[[3-[[2-Méthylimidazo[1,2-a]pyridin-8-yl]oxyméthyl]-2,4-dichlorophényl]-sulfonyl]-2(S)-[[4-[4-[(amino)(hydroxyimino)méthyl]benzoyl]pipérazin-1-yl]-carbonyl]pyrrolidine

En opérant de façon analogue à l'exemple 1, au départ du composé obtenu selon la préparation IX, on obtient le produit attendu sous forme de cristaux blancs (Rendement = 84 %).
F = 165-167°C
[α]²³_{D} = + 9,6° (c = 1,04 ; DMSO)

### PREPARATION X

### 1-[[3-[[2-Méthylimidazo[1,2-a]pyridin-8-yl]oxyméthyl]2,4-dichlorophényl]-sulfonyl]-2(S)-[[4-[4-[(acétoxyimino)(amino)méthyl]benzoyl]pipérazin-1-yl]-carbonyl]pyrrolidine

En opérant de façon analogue à la préparation VI, au départ du composé obtenu selon l'exemple 4, on obtient le produit attendu sous forme de cristaux blancs (Rendement = 98 %).
F = 115-118°C
[α]²³_{D} = + 10,2° (c = 1 ; DMSO)

### Exemple 5

### 1-[[3-[[2-Méthylimidazo[1,2-a]pyridin-8-yl]oxyméthyl]-2,4-dichlorophényl]-sulfonyl]-2(S)-[[4-[4-(aminoiminométhyl)benzoyl]pipérazin-1-yl]carbonyl]-pyrrolidine

En opérant de façon analogue à l'exemple 2, au départ du composé obtenu selon la préparation X, on obtient le produit attendu sous forme de cristaux jaune pâle (Rendement = 60 %).
F = 159-161°C
[α]²⁴_{D} = - 30° (c = 1,02 ; CH₃OH)

### Exemple 6

### 1-[[3-[[2-Méthylimidazo[1,2-a]pyridin-8-yl]oxyméthyl]-2,4-dichlorophényl]-sulfonyl]-2(S)-[[4-[4-(aminoiminométhyl)benzoyl]pipérazin-1-yl]carbonyl]-pyrrolidine, méthanesulfonate

En opérant de façon analogue à l'exemple 3, au départ du composé obtenu selon l'exemple 5, on obtient le produit attendu sous forme de cristaux blancs (Rendement = 93 %).
F = 172-174°C
[α]²⁶_{D} = - 19,3° (c = 1,01 ; CH₃OH)

### PREPARATION XI

### N-[[3-[[2,3-Diméthylimidazo[1,2-a]pyridin-8-yl]oxyméthyl]-2,4-dichlorophényl]sulfonyl]-L-proline, méthyl ester

En opérant de façon analogue à la préparation I, au départ de 2,3-diméthyl-8-hydroxy-imidazo[1,2-a]pyridine, on obtient le produit attendu sous forme de cristaux beiges (Rendement = 50 %).
F = 128-130°C
[α]²⁹_{D} = - 8,4° (c = 0,97 ; C₂H₅OH)

### PREPARATION XII

### N-[[3-[[2,3-Diméthylimidazo[1,2-a]pyridin-8-yl]oxyméthyl]-2,4-dichlorophényl]sulfonyl]-L-proline

En opérant de façon analogue à la préparation II, au départ du composé obtenu selon la préparation XI, on obtient le produit attendu sous forme de cristaux blanc cassé (Rendement = 60 %).
F = 140-145°C
[α]²²_{D} = + 15,8° (c = 0,99 ; C₂H₅OH)

### PREPARATION XIII

### 1-[[3-[[2,3-Diméthylimidazo[1,2-a]pyridin-8-yl]oxyméthyl]-2,4-dichlorophényl]-sulfonyl]-N-[3-[(4-cyanobenzoyl)amino]propyl]-2(S)-pyrrolidinecarboxamide

En opérant de façon analogue à la préparation V, au départ du composé obtenu selon la préparation XII, on obtient le produit attendu sous forme de cristaux beiges (Rendement = 62 %).
F = 128-130°C
[α]²⁶_{D} = - 7° (c = 0,95 ; CH₂Cl₂)

### Exemple 7

### 1-[[3-[[2,3-Diméthylimidazo[1,2-a]pyridin-8-yl]oxyméthyl]-2,4-dichlorophényl]-sulfonyl]-N-[3-[[4-[(amino)(hydroxylmino)méthyl]benzoyl]amino]propyl]-2(S)-pyrrolidinecarboxamide

En opérant de façon analogue à l'exemple 1, au départ du composé obtenu selon la préparation XIII, on obtient le produit attendu sous forme de cristaux blanc cassé (Rendement = 74 %).
F = 131-133°C
[α]²⁴_{D} = - 9,5° (c = 1,01 ; DMSO)

### PREPARATION XIV

### 1-[[3-[[2,3-Diméthylimidazo[1,2-a]pyridin-8-yl]oxyméthyl]-2,4-dichlorophényl]- sulfonyl]-N-[3-[[4-[(acétoxyimino)(amino)méthyl]benzoyl]amino]propyl]-2(S)-pyrrolidinecarboxamide

En opérant de façon analogue à la préparation VI, au départ du composé obtenu selon l'exemple 7, on obtient le produit attendu sous forme de cristaux blancs (Rendement = 98 %).
F = 140-142°C
[α]²²_{D} = - 7,5° (c = 1,00 ; DMSO)

### Exemple 8

### 1-[[3-[[2,3-Diméthylimidazo[1,2-a]pyridin-8-yl]oxyméthyl]-2,4-dichlorophényl]-sulfonyl]-N-[3-[[4-(iminoaminométhyl)benzoyl]amino]propyl]-2(S)-pyrrolidine-carboxamide

En opérant de façon analogue à l'exemple 2, au départ du composé obtenu selon la préparation XIV, on obtient le produit attendu sous forme de cristaux jaune pâle (Rendement = 53 %).
F = 150-152°C
[α]²⁵_{D} = - 15,5° (c = 1,1 ; CH₂Cl₂)

### Exemple 9

### 1-[[3-[[2,3-Diméthylimidazo[1,2-a]pyridin-8-yl]oxyméthyl]-2,4-dichlorophényl]sulfonyl]-N-[3-[[4-(aminoiminométhyl)benzoyl]amino]propyl]-2(S)-pyrrolidinecarboxamide, méthanesulfonate

En opérant de façon analogue à l'exemple 3, au départ du composé obtenu selon l'exemple 8, on obtient le produit attendu sous forme de cristaux blancs (Rendement = 82 %).
F = 179-181°C
[α]²³_{D} = - 39° (c = 1,02 ; CH₃OH)

### PREPARATION XV

### 1-[[3-[[2,3-Diméthylimidazo[1,2-a]pyridin-8-yl]oxyméthyl]-2,4-dichlorophényl]-sulfonyl]-2(S)-[[4-(4-cyanobenzoyl)pipérazin-1-yl]carbonyl]pyrrolidine

En opérant de façon analogue à la préparation IX, au départ du composé obtenu selon la préparation XII, on obtient le produit attendu sous forme de cristaux jaunes (Rendement = 41 %).
F = 125-128°C
[α]²⁶_{D} = + 0,9° (c = 0,85 ; CH₂Cl₂)

### Exemple 10

### 1-[[3-[[2,3-Diméthylimidazo[1,2-a]pyridin-8-yl]oxyméthyl]-2,4-dichlorophényl]-sulfonyl]-2(S)-[[4-[4-[(amino)(hydroxyimino)méthyl]benzoyl]pipérazin-1-yl]-carbonyl]pyrrolidine

En opérant de façon analogue à l'exemple 4, au départ du composé obtenu selon la préparation XV, on obtient le produit attendu sous forme de cristaux crème (Rendement = 76 %).
F = 179-181°C
[α]²⁶_{D} = - 0,7° (c = 1,01 ; CH₂Cl₂)

### PREPARATION XVI

### 1-[[3-[[2,3-Diméthylimidazo[1,2-a]pyridin-8-yl]oxyméthyl]-2,4-dichlorophényl]-sulfonyl]-2(S)-[[4-[4-[(acétoxyimino)(amino)méthyl]benzoyl]pipérazin-1-yl]-carbonyl]pyrrolidine

En opérant de façon analogue à la préparation X, au départ du composé obtenu selon l'exemple 10, on obtient le produit attendu sous forme de cristaux blancs (Rendement = 98 %).
F = 142-145°C
[α]²⁶_{D} = - 13,5° (c = 1,05 ; CH₂Cl₂)

### Exemple 11

### 1-[[3-[[2,3-Diméthylimidazo[1,2-a]pyridin-8-yl]oxyméthyl]-2,4-dichlorophényl]-sulfonyl]-2(S)-[[4-[4-(aminoiminométhyl)benzoyl]-pipérazin-1-yl]-carbonyl]-pyrrolidine

En opérant de façon analogue à l'exemple 5, au départ du composé obtenu selon la préparation XVI, on obtient le produit attendu sous forme de cristaux blancs (Rendement = 47 %).
F = 170-172°C
[α]²¹_{D} = - 2,9° (c = 1,00 ; CH₂Cl₂)

### Exemple 12

### 1-[[3-[[2,3-Diméthylimidazo[1,2-a]pyridin-8-yl]oxyméthyl]-2,4-dichlorophényl]- sulfonyl]-2(S)-[[4-[4-(aminoiminométhyl)benzoyl]-pipérazin-1-yl]carbonyl]-pyrrolidine, dichlorhydrate

On prépare une solution de 736 mg (1,03.10⁻³ mole) du composé obtenu selon l'exemple 11 dans 25 ml de dichlorométhane et on ajoute 650 µl (2,6.10⁻³ mole) d'une solution de chlorure d'hydrogène 4N dans le dioxane. Après 3 heures sous agitation, on sépare le précipité formé et on le reprend dans de l'éther diéthylique. On obtient ainsi un solide jaune pâle que l'on sépare par filtration. Après séchage sous vide, le produit est repris en solution dans de l'eau, filtré et lyophilisé. On obtient ainsi 710 mg du produit attendu sous forme d'un solide floconneux couleur crème (Rendement = 87 %).
F = 225-229°C
[α]²⁶_{D} = + 19,5° (c = 1,07; CH₃OH)

### Exemple 13

### 1-[[3-[[2,3-Diméthylimidazo[1,2-a]pyridin-8-yl]oxyméthyl]-2,4-dichlorophényl]-sulfonyl]-N-[4-(aminoiminométhyl)phénylméthyl]-2(S)-pyrrolidinecarboxamide

On prépare une solution de 5 g (10,1.10⁻³ mole) du composé obtenu selon la préparation XII dans 100 ml de diméthylformamide et on ajoute 2,12 g (11.10⁻³ mole) d'EDCI et 1,52 g (11.10⁻³ mole) de HOAT. Après une heure sous agitation à température ambiante, on ajoute cette solution à 2,5 g (11.10⁻³ mole) de dichlorhydrate de 4-(aminométhyl)-benzènecarboximidamide [ou 4-(aminoimino-méthyl)-benzylamine] en solution dans 100 ml de DMF et 1,2 ml (11.10⁻³ mole) de N-méthylmorpholine. On laisse sous agitation pendant 15 heures à température ambiante. Le milieu réactionnel est filtré et le filtrat est versé sur l'éther éthylique sous agitation. On sépare le précipité ainsi formé et on le reprend en solution dans de l'eau. On ajoute du dichlorométhane et une solution 2N d'hydroxyde de sodium de façon à amener le milieu à pH13. On sépare les phases aqueuses et organiques et on lave la phase organique avec de l'eau. Après séchage sur sulfate de magnésium, la phase organique est concentrée sous pression réduite. Le produit brut obtenu est purifié par chromatographie sur gel de silice greffé NH₂ avec pour éluant un mélange dichlorométhane/éthanol (95/5 ; v/v). On obtient ainsi 3,6 g du produit attendu sous forme d'un solide blanc (Rendement = 57 %).
F = 144-146°C
[α]²¹_{D} = - 34,5° (c = 1,03 ; CHCl₃)

### Exemple 14

### 1-[[3-[[2,3-Diméthylimidazo[1,2-a]pyridin-8-yl]oxyméthyl]-2,4-dichlorophényl]-sulfonyl]-N-[4-(aminoiminométhyl)phénylméthyl]-2(S)-pyrrolidinecarboxamide, chlorhydrate

On prépare une solution de 0,42 g (0,66.10⁻³ mole) du composé obtenu selon l'exemple 13 dans 100 ml d'éther diéthylique et on ajoute, sous agitation, 0,75 ml d'une solution 1N de chlorure d'hydrogène dans l'éther diéthylique. les cristaux formés sont séparés par filtration, lavés avec de l'éther et séchés sous vide. On obtient ainsi le produit attendu sous forme de cristaux blancs (Rendement = 81 %).
F = 210-214°C
[α]²²_{D} = - 12 ° (c = 1,00 ; C₂H₅OH)

### PREPARATION XVII

### N-[[3-[[3-Bromo-2-méthylimidazo[1,2-a]pyridin-8-yl]oxyméthyl]-2,4-dichlorophényl]sulfonyl]-L-proline, méthyl ester

On prépare une solution de 7 g (14.10⁻³ mole) du composé obtenu selon la préparation I dans un mélange de 100 ml de dioxane et 100 ml d'éthanol et on ajoute par portions, sous agitation et à température ambiante, 2,5 g (14.10⁻³ mole) de N-bromosuccinimide. Après agitation pendant 1 heure, on chasse les solvants sous pression réduite. La solution du résidu d'évaporation repris dans du dichlorométhane est lavée à l'eau puis séchée sur sulfate de magnésium et concentrée sous pression réduite. Après purification par chromatographie sur gel de silice avec pour éluant le mélange toluène/2-propanol (97/3 ; v/v) et recristallisation dans le 2-propanol, on obtient 7,3 g du produit attendu (Rendement = 90 %).
F = 146°C
[α]²³_{D} = - 17° (c = 1,5 ; CH₂Cl₂)

### PREPARATION XVIII

### N-[[3-[[3-Bromo-2-méthylimidazo[1,2-a]pyridin-8-yl]oxyméthyl]-2,4-dichlorophényl]sulfonyl]-L-proline

En opérant de façon analogue à la préparation II, au départ du composé obtenu selon la préparation XVII, on obtient le produit attendu sous forme d'un solide poudreux crème (Rendement = 98 %).
F = 145°C
[α]²⁰_{D} = - 30,5° (c = 1,05 ; CH₂Cl₂)

### PREPARATION XIX

### 1-[[3-[[3-Bromo-2-méthylimidazo[1,2-a]pyridin-8-yl]oxyméthyl]-2,4-dichlorophényl]sulfonyl]-N-[3-[1,1-diméthyléthoxycarbonylamino]propyl]-2(S)-pyrro lidinecarboxamide

En opérant de façon analogue à la préparation V, au départ du composé obtenu selon la préparation XVIII et de l'ester 1,1-diméthyléthylique de l'acide (3-aminopropyl)carbamique [ou N-Boc-propanediamine], on obtient le produit attendu sous forme d'un solide blanc (Rendement = 95 %).
F = 65°C
[α]²²_{D} = - 43° (c = 0,44 ; CH₃OH)

### PREPARATION XX

### 1-[[3-[[3-Bromo-2-méthylimidazo[1,2-a]pyridin-8-yl]oxyméthyl]-2,4-dichlorophényl]sulfonyl]-N-[3-aminopropyl]-2(S)-pyrrolidinecarboxamide

On prépare une solution de 0,6 g (0,83.10⁻³ mole) du composé obtenu selon la préparation XIX, dans 10 ml de dichlorométhane et on ajoute, à 0°C, 89 mg (0,83.10⁻³ mole) d'anisole et 3 ml d'acide trifluoroacétique. Le mélange réactionnel est ensuite maintenu sous agitation pendant 4 heures à température ambiante puis on chasse le solvant sous pression réduite. Le résidu est repris dans 50 ml d'eau, la phase aqueuse obtenue est amenée à pH10 à l'aide d'une solution 1N d'hydroxyde de sodium, puis extraite par du dichlorométhane. La phase organique est lavée à l'eau, séchée sur sulfate de sodium puis concentrée sous pression réduite. On obtient ainsi 0,45 g du produit attendu sous forme d'un solide blanc poudreux.
F = 75°C
[α]²²_{D} = - 72° (c = 0,40 ; CH₃OH)

### Exemple 15

### 1-[[3-[[3-Bromo-2-méthylimidazo[1,2-a]pyridin-8-yl]oxyméthyl]-2,4-dichlorophényl]sulfonyl]-N-[3-[4-(aminoiminométhyl)benzoylamino]propyl]-2(S)-pyrrolidinecarboxamide

On prépare une suspension de 80 mg (0,4.10⁻³ mole) du chlorhydrate de l'acide 4-(aminoiminométhyl)benzoïque dans 6 ml de DMF et on ajoute 84 mg (0,44.10⁻³ mole) d'EDCI, et 60 mg (0.44.10⁻³ mole) de HOAT. On laisse sous agitation à température ambiante pendant 10 mn puis on ajoute 250 mg (0,40.10⁻³ mole) du composé obtenu selon la préparation XX. On agite le mélange réactionnel pendant 20 heures à température ambiante puis on le verse sur de l'eau. On extrait avec du dichlorométhane, puis on lave la phase organique ainsi obtenue avec une solution saturée de bicarbonate de sodium, puis avec de l'eau. On sèche sur sulfate de sodium et on concentre sous pression réduite. Le produit brut est purifié par chromatographie sur gel de silice greffé NH₂ avec pour éluant un mélange dichlorométhane/méthanol (98/2 ; v/v). On obtient ainsi 170 mg du produit attendu sous forme d'un solide blanc (Rendement = 55 %).
F = 170°C
[α]²²_{D} = - 2,6° (c = 0,16 ; CH₃OH)

### Exemple 16

### 1-[[3-[[3-Bromo-2-méthylimidazo[1,2-a]pyridin-8-yl]oxyméthyl]-2,4-dichloro-phényl]sulfonyl]-N-[3-[4-(aminoiminométhyl)benzoylamino]propyl]-2(S)-pyrrolidinecarboxamide, chlorhydrate

On prépare une solution de 153 mg (0,2.10⁻³ mole) du composé obtenu selon l'exemple 15, dans 8 ml d'acétate d'éthyle et 2 ml d'éthanol et on ajoute 0,5 ml d'une solution saturée de chlorure d'hydrogène dans l'éther diéthylique. On agite le mélange pendant 10 mn puis on élimine les solvants sous pression réduite. On reprend ensuite le résidu en solution dans l'eau, filtre et lyophilise le filtrat. On obtient ainsi 120 mg du produit attendu sous forme d'une poudre blanche (Rendement = 75 %).
F = 200°C
[α]²²_{D} = - 13° (c = 0,39 ; CH₃OH)

### PREPARATION XXI

### 1-[[3-[[3-Bromo-2-méthyl-imidazo[1,2-a]pyridin-8-yl]oxyméthyl]-2,4-dichlorophényl]sulfonyl]-2(S)-[[4-(1,1-diméthyléthoxycarbonyl)pipérazin-1-yl]carbonyl]-pyrrolidine

En opérant de façon analogue à la préparation XIX, au départ de l'ester 1,1-diméthyléthylique de l'acide 1-pipérazinecarboxylique, on obtient le produit attendu sous forme de cristaux blancs (Rendement = 90 %).
F = 85°C
[α]²²_{D} = + 9,6° (c = 1,00 ; DMSO)

### PREPARATION XXII

### 1-[[3-[[3-Bromo-2-méthylimidazo[1,2-a]pyridin-8-yl]oxyméthyl]-2,4-dichlorophényl]sulfonyl]-2(S)-[[pipérazin-1-yl]carbonyl]pyrrolidine

En opérant de façon analogue à la préparation XX, au départ du composé obtenu selon la préparation XXI, on obtient le produit attendu sous forme de cristaux blancs (Rendement = 90 %).
F = 178-180°C
[α]²¹_{D} = + 16,5° (c = 1,01 ; CH₃OH)

### Exemple 17

### 1-[[3-[[3-Bromo-2-méthylimidazo[1,2-a]pyridin-8-yl]oxyméthyl]-2,4-dichlorophényl]sulfonyl]-2(S)-[[4-[4-(aminoiminométhyl)benzoyl]pipérazin-1-yl]-carbonyl]pyrrolidine, chlorhydrate

En opérant de façon analogue à l'exemple 15, au départ du composé obtenu selon la préparation XXII, et après purification par chromatographie en phase inverse sur gel de silice (gel de silice greffé, commercialisé sous la nomenclature de RP18), avec pour éluant un mélange acétonitrile/eau/acide chlorhydrique (75/25/1 ; v/v/v), on obtient le produit attendu sous forme de cristaux jaune pâle (Rendement = 40 %).
F = 200-204°C
[α]¹⁹_{D} = + 21° (c = 1,02 ; CH₃OH)

### Exemple 18

### 1-[[3-[[3-Bromo-2-méthylimidazo[1,2-a]pyridin-8-yl]oxyméthyl]-2,4-dichlorophényl]sulfonyl]-N-[4-(aminoiminométhyl)phénylméthyl]-2(S)-pyrrolidine-carboxamide

On prépare une solution de 1,13 g (2.10⁻³ mole) du composé obtenu selon la préparation XVIII dans 10 ml de DMF et on ajoute 382 mg (2.10⁻³ mole) de EDCI et 272 mg (2.10⁻³ mole) de HOAT. La solution est agitée pendant 20 mn à température ambiante puis ajoutée doucement à un mélange de 453 mg (2.10⁻³ mole) du dichlorhydrate de 4-(aminométhyl)benzènecarboximidamide et de 202 mg (2.10⁻³ mole) de N-méthylmorpholine dans 10 ml de DMF. Le mélange réactionnel est agité pendant 20 heures à température ambiante puis versé dans 150 ml d'eau. On ajoute une solution 1N d'hydroxyde de sodium sous agitation de façon à amener le milieu à pH13, puis on extrait par du dichlorométhane. La phase organique est lavée puis séchée sur sulfate de magnésium et concentrée sous pression réduite. Le produit obtenu est purifié par chromatographie sur gel de silice (gel de silice greffé NH₂) avec pour éluant un mélange dichlorométhane/méthanol (95/5 ; v/v). On obtient ainsi 280 mg du produit attendu sous forme d'un solide blanc (Rendement = 24 %).
F = 124°C
[α]²⁵_{D} = - 30° (c = 0,57 ; CH₃OH)

### Exemple 19

### 1-[[3-[[3-Bromo-2-méthylimidazo[1,2-a]pyridin-8-yl]oxyméthyl]-2,4-dichlorophényl]sulfonyl]-N-[4-(aminoiminométhyl)phénylméthyl]-2(S)-pyrrolidine-carboxamide, chlorhydrate

En opérant de façon analogue à l'exemple 16, au départ du composé obtenu selon l'exemple 18, on obtient le produit attendu sous forme d'un solide blanc (Rendement = 92 %).
F = 214°C
[α]²⁵_{D} = - 27° (c = 0,68 ; CH₃OH)

### PREPARATION XXIII

### N-[[3-[(2-Méthylquinoxalin-8-yl)oxyméthyl]-2,4-dichlorophényl]sulfonyl]-L-proline, méthyl ester

En opérant de façon analogue à la préparation I, au départ de 8-hydroxy-2-méthylquinoxaline, on obtient le produit attendu sous forme de cristaux blancs (Rendement = 92 %).
F = 158-159°C
[α]²⁴_{D} = - 24,5° (c = 1,00 ; CHCl₃)

### PREPARATION XXIV

### N-[[3-[(2-Méthylquinoxalin-8-yl)oxyméthyl]-2,4-dichlorophényl]sulfonyl]-L-proline

En opérant de façon analogue à la préparation II, au départ du produit obtenu selon la préparation XXIII, on obtient le produit attendu sous forme de cristaux blanc cassé (Rendement = 90 %).
F = 238-240°C
[α]²⁶_{D} = - 111° (c = 0,97 ; CHCl₃)

### PREPARATION XXV

### 1-[[3-[(2-méthylquinoxalin-8-yl)oxyméthyl]-2,4-dichlorophényl]sulfonyl]-N-[3-[(4-cyanobenzoyl)amino]propyl]-2(S)-pyrrolidinecarboxamide

En opérant de façon analogue à la préparation V, au départ du produit obtenu selon la préparation XXIV, on obtient le produit attendu sous forme de cristaux blancs (Rendement = 79 %).
F = 104-106°C
[α]²⁶_{D} = - 32° (c = 0,96 ; CHCl₃)

### Exemple 20

### 1-[[3-[(2-Méthylquinoxalin-8-yl)oxyméthyl]-2,4-dichlorophényl]sulfonyl]-N-[3-[[4-[(amino)(hydroxyimino)méthyl]benzoyl]amino]propyl]-2(S)-pyrrolidine- carboxamide

En opérant de façon analogue à l'exemple 1, au départ du produit obtenu selon la préparation XXV, on obtient le produit attendu sous forme de cristaux blancs (Rendement = 88 %).
F = 172-176°C
[α]²³_{D} = - 7,5° (c = 0,98 ; DMSO)

### PREPARATION XXVI

### 1-[[3-[(2-Méthylquinoxalin-8-yl)oxyméthyl]-2,4-dichlorophényl]sulfonyl]-N-[3-[[4-[(acétoxyimino)(amino)méthyl]benzoyl]amino]propyl]-2(S)-pyrrolidine carboxamide

En opérant de façon analogue à la préparation VI, au départ du composé obtenu selon l'exemple 20, on obtient le produit attendu sous forme de cristaux rosés (Rendement = 95 %).
F = 151-154°C
[α]²⁴_{D} = - 10° (c = 0,95 ; DMSO)

### Exemple 21

### 1-[[3-[(2-Méthylquinoxalin-8-yl)oxyméthyl]-2,4-dichlorophényl]sulfonyl]-N-[3-[[4-(aminoiminométhyl)benzoyl]amino]propyl]-2(S)-pyrrolidinecarboxamide

En opérant de façon analogue à l'exemple 2, au départ du composé obtenu selon la préparation XXVI, on obtient le produit attendu sous forme de cristaux rosés (Rendement = 55 %).
F = 136-140°C
[α]²⁴_{D} = - 10° (c = 1,00 ; DMSO)

### Exemple 22

### 1-[[3-[(2-Méthylquinoxalin-8-yl)oxyméthyl]-2,4-dichlorophényl]sulfonyl]-N-[3-[[4-(aminoiminométhyl)benzoyl]amino]propyl]-2(S)-pyrrolidinecarboxamide, méthanesulfonate

En opérant de façon analogue à l'exemple 3, au départ du composé obtenu selon l'exemple 21, on obtient le produit attendu, sous forme de cristaux jaune clair (Rendement = 84 %).
F = 165-167°C
[α]²⁵_{D} = - 38,2° (c = 1,03 ; CH₃OH)

### PREPARATION XXVII

### 1-[[3-[(2-Méthylquinoxalin-8-yl)oxyméthyl]-2,4-dichlorophényl]sulfonyl]-2(S)-[[4-(4-cyanobenzoyl)pipérazin-1-yl]carbonyl]pyrrolidine

En opérant de façon analogue à la préparation IX, au départ du composé obtenu selon la préparation XXIV, on obtient le produit attendu sous forme de cristaux blancs (Rendement = 96 %).
F = 126-130°C
[α]²⁶_{D} = - 0,4° (c = 1,13 ; CHCl₃)

### Exemple 23

### 1-[[3-[(2-Méthylquinoxalin-8-yl)oxyméthyl]-2,4-dichlorophényl]sulfonyl]-2(S)-[[4-[4-[(amino)(hydroxyimino)méthyl]benzoyl]pipérazin-1-yl]carbonyl]- pyrrolidine

En opérant de façon analogue à l'exemple 4, au départ du composé obtenu selon la préparation XXVII, on obtient le produit attendu sous forme de cristaux blancs (Rendement = 86 %).
F = 210-212°C
[α]²⁴_{D} = + 19,5° (c = 0,47 ; DMSO)

### PREPARATION XXVIII

### 1-[[3-[(2-Méthylquinoxalin-8-yl)oxyméthyl]2,4-dichlorophényl]sulfonyl]-2(S)-[[4-[4-[(acétoxyimino)(amino)méthyl]benzoyl]pipérazin-1-yl]carbonyl]- pyrrolidine

En opérant de façon analogue à la préparation X, au départ du composé obtenu selon l'exemple 23, on obtient le produit attendu sous forme de cristaux jaunes (Rendement = 98 %).
F = 161-165°C
[α]²³_{D} = + 9° (c = 1,02 ; DMSO)

### Exemple 24

### 1-[[3-[(2-Méthylquinoxalin-8-yl)oxyméthyl]-2,4-dichlorophényl]sulfonyl]-2(S)-[[4-[4-(aminoiminométhyl)benzoyl]-pipérazin-1-yl]carbonyl]pyrrolidine

En opérant de façon analogue à l'exemple 5, au départ du composé obtenu selon la préparation XXVIII, on obtient le produit attendu sous forme de cristaux beiges (Rendement = 45 %).
F = 155-158°C
[α]²⁴_{D} = + 9,7° (c = 0,95 ; DMSO)

### Exemple 25

### 1-[[3-[(2-Méthylquinoxalin-8-yl)oxyméthyl]-2,4-dichlorophényl]sulfonyl]-2(S)-[[4-[4-(aminoiminométhyl)benzoyl]-pipérazin-1-yl]carhonyl]pyrrolidine, chlorhydrate

En opérant de façon analogue à l'exemple 16, au départ du composé obtenu selon l'exemple 24, on obtient le produit attendu sous forme d'un solide blanc (Rendement = 95 %).
F = 192-195°C
[α]²⁴_{D} = + 6,7° (c = 1,03 ; DMSO)

### Exemple 26

### 1-[[3-[(2-Méthylquinoxalin-8-yl)oxyméthyl]-2,4-dichlorophényl]sulfonyl]-N-[4-(aminoiminométhyl)phénylméthyl]-2(S)-pyrrolidinecarboxamide

En opérant de façon analogue à l'exemple 13, au départ du composé obtenu selon la préparation XXIV, on obtient le produit attendu sous forme d'un solide beige (Rendement = 27 %).
F = 120-125°C
[α]²³_{D} = - 42° (c = 1,00 ; CHCl₃)

### Exemple 27

### 1-[[3-[(2-Méthylquinoxalin-8-yl)oxyméthyl]-2,4-dichlorophényl]sulfonyl]-N-[4-(aminoiminométhyl)phénylméthyl]-2(S)-pyrrolidinecarboxamide, chlorhydrate

On prépare une solution de 387 mg (0,62.10⁻³ mole) du composé obtenu selon l'exemple 26, dans 100 ml d'éther éthylique et on ajoute 0,7 ml d'une solution 1N de chlorure d'hydrogène dans l'éther éthylique. Les cristaux formés sont filtrés, lavés à l'éther, séchés sous vide et remis en solution dans 10 ml d'eau. Après filtration et lyophilisation du filtrat, on obtient 376 mg du produit attendu sous forme d'une poudre blanche (Rendement = 92 %).-F = 180-184°C
[α]²³_{D} = - 43° (c = 0,99 ; éthanol)

### PREPARATION XXIX

### N-[[3-[[2-Méthyl-4H-pyrido[1,2-a]pyrimidin-9-yl-4-one]oxyméthyl]-2,4-dichlorophényl]sulfonyl]-(L)-proline, méthyl ester

En opérant de façon analogue à la préparation I, au départ de 9-hydroxy-2-méthyl-4H-pyrido[1,2-a]pyrimidin-4-one, on obtient le produit attendu sous forme de cristaux blanc cassé (Rendement = 56 %).
F = 166°C
[α]²²_{D} = - 24° (c = 0,37 ; CHCl₃)

### PREPARATION XXX

### N-[[3-[[2-méthyl-4H-pyrido[1,2-a]pyrimidin-9-yl-4-one]oxyméthyl]-2,4-dichlorophényl]sulfonyl]-(L)-proline

En opérant de façon analogue à la préparation II, au départ du composé obtenu selon la préparation XXIX, on obtient le produit attendu sous forme d'un solide beige (Rendement = 92 %).
F = 150°C
[α]²²_{D} = - 86° (c = 0,4 ; CHCl₃)

### PREPARATION XXXI

### 1-[[3-[[2-Méthyl-4H-pyrido[1,2-a]pyrimidin-9-yl-4-one]oxyméthyl]-2,4-dichlorophényl]sulfonyl]-N-[3-[(4-cyanobenzoyl)amino]propyl]-2(S)-pyrrolidine-carboxamide

En opérant de façon analogue à la préparation V, au départ du composé obtenu selon la préparation XXX, on obtient le produit attendu sous forme d'un solide blanc (Rendement = 83 %).
F = 112°C
[α]²²_{D} = - 50° (c = 0,31 ; CHCl₃)

### Exemple 28

### 1-[[3-[[2-Méthyl-4H-pyrido[1,2-a]pyrimidin-9-yl-4-one]oxyméthyl]-2,4-dichlorophényl]sulfonyl]-N-[3-[4-[(amino)(hydroxyimino)méthyl]benzoylamino]propyl]-2(S)-pyrrolidinecarboxamide

En opérant de façon analogue à l'exemple 1, au départ du composé obtenu selon la préparation XXXI, on obtient le produit attendu sous forme d'un solide blanc (Rendement = 87 %).
F = 251°C
[α]²²_{D} = - 11° (c = 0,36 ; DMSO)

### PREPARATION XXXII

### 1-[[3-[[2-méthyl-4H-pyrido[1,2-a]pyrimidin-9-yl-4-one]oxyméthyl]-2,4-dichlorophényl]sulfonyl]-N-[3-[4-[(acétoxyimino)(amino)méthyl]benzoylamino]propyl]-2(S)-pyrrolidinecarboxamide

En opérant de façon analogue à la préparation VI, au départ du composé obtenu selon l'exemple 28, on obtient le produit attendu sous forme d'une poudre blanc-crème (Rendement = 95 %).
F = 150°C
[α]²²_{D} = - 24,5° (c = 0,33 ; CHCl₃)

### Exemple 29

### 1-[[3-[[2-Méthyl-4H-pyrido[1,2-a]pyrimidin-9-yl-4-one]oxyméthyl]-2,4-dichlorophényl]sulfonyl]-N-[3-[[4-(aminoiminométhyl)benzoyl]amino]propyl]-2(S)-pyrrolidinecarboxamide

En opérant de façon analogue à l'exemple 2, au départ du composé obtenu selon la préparation XXXII, on obtient le produit attendu sous forme d'un solide poudreux blanc (Rendement = 62 %).
F = 153°C
[α]²²_{D} = - 19° (c = 0,32 ; DMSO)

### Exemple 30

### 1-[[3-[[2-Méthyl-4H-pyrido[1,2-a]pyrimidin-9-yl-4-one]oxyméthyl]-2,4-dichlorophényl]sulfonyl]-N-[3-[[4-(aminoiminométhyl)benzoyl]amino]propyl]-2(S)-pyrrolidinecarboxamide, bis(méthanesulfonate)

On prépare une solution de 160 mg (0,224.10⁻³ mole) du composé obtenu selon l'exemple 29 dans 3 ml de dichlorométhane et 3 ml d'éthanol. On ajoute 43 mg (0,45.10⁻³ mole) d'acide méthanesulfonique et on maintient le mélange sous agitation pendant 10 mn. On ajoute ensuite 20 ml d'éther diéthylique et, après 5 mn, on filtre les cristaux formés et on les lave sur le filtre avec de l'éther. Après séchage sous vide, les cristaux sont remis en solution dans 20 ml d'eau et lyophilisés. On obtient ainsi 170 mg du produit attendu sous forme de fins cristaux blancs (Rendement = 85 %).
F = 183°C
[α]²⁴_{D} = - 32° (c = 0,46 ; CH₃OH)

### PREPARATION XXXIII

### 1-[4-Cyanobenzoyl]-pipérazine, trifluoroacétate

On prépare une solution de 40,7 g (129.10⁻³ mole) du composé obtenu selon la préparation VII dans 500 ml de dichlorométhane et on ajoute progressivement 150 ml d'acide trifluoroacétique. Le mélange est ensuite agité à température ambiante pendant 30 mn, puis concentré sous pression réduite. Le résidu est repris sous agitation dans 400 ml d'éther diéthylique ; les cristaux formés sont séparés par filtration puis séchés sous vide. On obtient ainsi 30,8 g du produit attendu sous forme de cristaux blancs (Rendement = 87 %).
F = 180°C

### PREPARATION XXXIV

### 1-(1,1-Diméthyléthoxycarbonyl)-2(S)-[[4-(4-cyanobenzoyl)pipérazin-1-yl]-carbonyl]pyrrolidine

On prépare une solution de 10,78 g (50.10⁻³ mole) de N-(1,1-diméthyl-éthoxycarbonyl)-L-proline (ou N-Boc-L-proline) dans 90 ml de DMF et on ajoute 11,47 g (60.10⁻³ mole) de EDCI et 8,18 g (60.10⁻³ mole) de HOAT. On maintient le mélange sous agitation pendant 30 mn à température ambiante puis on ajoute une solution de 15 g (55.10⁻³ mole) du composé obtenu selon la préparation XXXIII dans 90 ml de DMF et 6,08 g (60.10⁻³ mole) de triéthylamine. Après agitation pendant 3 heures à température ambiante, le milieu réactionnel est versé sur de l'eau glacée et extrait par du dichlorométhane. La phase organique est lavée puis séchée sur sulfate de magnésium et concentrée sous pression réduite. Le produit brut est purifié par chromatographie sur gel de silice avec pour éluant un mélange dichlorométhane/méthanol (98/2 ; v/v). On obtient ainsi 20 g du produit attendu sous forme d'un solide blanc (Rendement : 97 %).
F = 50°C
[α]²³_{D} = + 3° (c = 0,50 ; CHCl₃)

### PREPARATION XXXV

### 2(S)-[4-(4-Cyanobenzoyl)pipérazin-1-yl-carbonyl]pyrrolidine, trifluoroacétate

En opérant de façon analogue à la préparation XX, au départ du composé obtenu selon la préparation XXXIV, on obtient le produit attendu sous forme d'un solide blanc (Rendement = 81 %).
F = 50°C
[α]²²_{D} = - 35° (c = 0,58 ; CHCl₃)

### PREPARATION XXXVI

### 1-[(3-Bromométhyl-2,4-dichlorophényl)sulfonyl]-2(S)-[[4-(4-cyanobenzoyl)-pipérazin-1-yl]carbonyl]pyrrolidine

On prépare une solution de 13,4 g (39,5.10⁻³ mole) de chlorure de 3-bromométhyl-2,4-dichlorobenzènesulfonyle dans 35 ml d'acétonitrile et on ajoute, à température ambiante, 16,9 g (39,5.10⁻³ mole) du composé obtenu selon la préparation XXXV. On ajoute ensuite goutte à goutte une solution de 10 g (0,1 mole) de bicarbonate de potassium dans 40 ml d'eau. Le mélange réactionnel est agité pendant 20 heures puis on ajoute de l'eau et extrait avec de l'acétate d'éthyle. La phase organique obtenue est lavée à l'eau, séchée sur sulfate de magnésium et concentrée sous pression réduite. Le produit brut est purifié par chromatographie sur gel de silice avec pour éluant un mélange dichlorométhane/acétate d'éthyle (9/1 ; v/v). On obtient ainsi 18,8 g du produit attendu (qui contient en partie son analogue chloré) sous forme d'un solide beige.
F = 115°C

### PREPARATION XXXVII

### 1-[[3-[[2-Méthylpyrido[1,2-a]pyrimidin-9-yl-4-one]oxyméthyl]-2,4-dichlorophényl]sulfonyl]-2(S)-[[4-(4-cyanobenzoyl)pipérazin-1-yl]carbonyl]pyrrolidine

En opérant de façon analogue à la préparation I, au départ de 9-hydroxy-2-méthylpyrido[1,2-a]pyrimidin-4-one et du composé obtenu selon la préparation XXXVI, on obtient après recristallisation dans un mélange acétate d'éthyle/diisopropyl éther, le produit attendu sous forme de cristaux blancs (Rendement = 62 %).
F = 160°C
[α]²²_{D} = + 9° (c = 0,39 ; CHCl₃)

### Exemple 31

### 1-[[3-[[2-Méthylpyrido[1,2-a]pyrimidin-9-yl-4-one]oxyméthyl]-2,4-dichlorophényl]sulfonyl]-2(S)-[[4-[4-[(amino)(hydroxyimino)méthyl]-benzoyl]pipérazin-1-yl]carbonyl]pyrrolidine

En opérant de façon analogue à l'exemple 1, au départ du composé obtenu selon la préparation XXXVII, on obtient le produit attendu sous forme d'un solide blanc (Rendement = 77 %).
F = 180°C
[α]²²_{D} = + 26° (c = 0,36 ; CHCl₃)

### PREPARATION XXXVIII

### 1-[[3-[[2-Méthylpyrido[1,2-a]pyrimidin-9-yl-4-one]oxyméthyl]-2,4-dichlorophényl]sulfonyl]-2(S)-[[4-[4[(acétoxyimino)(amino)méthyl]benzoyl]pipérazin-1-yl]carbonyl]pyrrolidine

En opérant de façon analogue à la préparation VI, au départ du composé obtenu selon l'exemple 31, on obtient le produit attendu sous forme d'un solide blanc (Rendement = 93 %).
F = 160°C
[α]²²_{D} = + 6° (c = 0,37 ; DMSO)

### Exemple 32

### 1-[[3-[[2-Méthylpyrido[1,2-a]pyrimidin-9-yl-4-one]oxyméthyl]-2,4-dichlorophényl]sulfonyl]-2(S)-[[4-[4-(aminoiminométhyl]benzoyl]pipérazin-1-yl]carbo nyl]pyrrolidine

En opérant de façon analogue à l'exemple 2, au départ du composé obtenu selon la préparation XXXVIII, on obtient le produit attendu sous forme d'un solide jaune (Rendement = 44 %).
F = 168°C
[α]²²_{D} = + 32° (c = 0,38 ; CHCl₃)

### Exemple 33

### 1-[[3-[[2-Méthylpyrido[1,2-a]pyrimidin-9-yl-4-one]oxyméthyl]-2,4-dichlorophényl]sulfonyl]-2(S)-[[4-[4-(aminoiminométhyl)benzoyl]pipérazin-1-yl]carbonyl]pyrrolidine, bis(méthanesulfonate)

En opérant de façon analogue à l'exemple 30, au départ du composé obtenu selon l'exemple 32, on obtient le produit attendu sous forme d'un solide blanc (Rendement = 80 %).
F = 181°C
[α]²⁴_{D} = + 13° (c = 0,35 ; CH₃OH)

### PREPARATION XXXIX

### N-[[3-[(2,4-Diméthylquinazolin-8-yl)oxyméthyl]-2,4-dichlorophényl]sulfonyl]-L-proline, méthyl ester

En opérant de façon analogue à la préparation I, au départ de 2,4-diméthyl-8-hydroxy-quinazoline, on obtient le produit attendu sous forme d'un solide blanc (Rendement = 70 %).
F = 140-142°C
[α]²⁴_{D} = - 29° (c = 1 ; CHCl₃)

### PREPARATION XL

### N-[[3-[(2,4-Diméthylquinazolin-8-yl)oxyméthyl]-2,4-dichlorophényl]sulfonyl]-L-proline,

En opérant de façon analogue à la préparation II, au départ du composé obtenu à la préparation XXXIX, on obtient le produit attendu sous forme d'un solide beige (Rendement = 98 %).
F = 94-96°C

### Exemple 34

### 1-[[3-[(2,4-Diméthylquinazolin-8-yl)oxyméthyl]-2,4-dichlorophényl]sulfonyl]-N-[4-(aminoiminométhyl)phénylméthyl]-2(S)-pyrrolidinecarboxamide

En opérant de façon analogue à l'exemple 13, au départ du composé obtenu à la préparation XL, on obtient le produit attendu sous forme de poudre blanche (Rendement = 25 %).
F = 139-142°C

### Exemple 35

### 1-[[3-[(2,4-Diméthylquinazolin-8-yl)oxyméthyl]-2,4-dichlorophényl]sulfonyl]-N-[4-(aminoiminométhyl)phénylméthyl]-2(S)-pyrrolidinecarboxamide, chlorhydrate

En opérant de façon analogue à l'exemple 16, au départ du composé obtenu selon l'exemple 34, on obtient le produit attendu sous forme de poudre blanche (Rendement = 95 %).
F = 177-179°C
[α]²¹_{D} = - 43° (c = 0,80 ; CH₃OH)

### PREPARATION XLI

### N-[[3-[[3-Chloro-2-méthylimidazo[1,2-a]pyridin-8-yl]oxyméthyl]-2,4-dichlorophényl]sulfonyl]-L-proline, méthyl ester

En opérant de façon analogue à la préparation XVII, en remplaçant le N-bromosuccinimide par le N-chlorosuccinimide, on obtient le produit attendu sous forme d'un solide beige (Rendement = 95 %).
F = 60°C
[α]²²_{D} = + 13° (c = 0,35 ; DMSO)

### PREPARATION XLII

### N-[[3-[[3-Chloro-2-méthylimidazo[1,2-a]pyridin-8-yl]oxyméthyl]-2,4-dichlorophényl]sulfonyl]-L-proline

En opérant de façon analogue à la préparation XVIII, au départ du composé obtenu selon la préparation XLI, on obtient le produit attendu sous forme d'un solide blanc (Rendement = 88 %).
F = 144°C
[α]²²_{D} = - 60,5° (c = 0,35 ; CHCl₃)

### PREPARATION XLIII

### 1-[[3-[[3-Chloro-2-méthylimidazo[1,2-a]pyridin-8-yl]oxyméthyl]-2,4-dichlorophényl]sulfonyl]-N-[3-[(4-cyanobenzoyl)amino]propyl]-2(S)-pyrrolidinecarboxamide

En opérant de façon analogue à la préparation V, au départ du composé obtenu à la préparation XLII, on obtient le produit attendu sous forme d'un solide blanc-cassé (Rendement = 51 %).
F = 115°C
[α]²²_{D} = - 32° (c = 0,60 ; CHCl₃)

### Exemple 36

### 1-[[3-[[3-Chloro-2-méthylimidazo[1,2-a]pyridin-8-yl]oxyméthyl]-2,4-dichlorophényl]sulfonyl]-N-[3-[[4-[(amino)(hydroxyimino)méthyl]benzoyl]amino]-propyl]-2(S)-pyrrolidinecarboxamide

En opérant de façon analogue à l'exemple 1, au départ du composé obtenu à la préparation XLIII, on obtient le produit attendu sous forme d'un solide blanc (Rendement = 96 %).
F = 155°C
[α]²²_{D} = - 41° (c = 0,40 ; CHCl₃)

### PREPARATION XLIV

### 1-[[3-[[3-Chloro-2-méthylimidazo[1,2-a]pyridin-8-yl]oxyméthyl]-2,4-dichlorophényl]sulfonyl]-N-[3-[[4-[(acétoxyimino)(amino)méthyl]benzoyl]amino]-propyl]-2(S)-pyrrolidinecarboxamide

En opérant de façon analogue à la préparation VI, au départ du composé obtenu selon l'exemple 36, on obtient le produit attendu sous forme d'un solide écru (Rendement = 82 %).
F = 170°C
[α]²¹_{D} = - 32° (c = 0,35 ; CHCl₃)

### Exemple 37

### 1-[[3-[[3-Chloro-2-méthylimidazo[1,2-a]pyridin-8-yl]oxyméthyl]-2,4-dichlorophényl]sulfonyl]-N-[3-[[4-(aminoiminométhyl)benzoyl]amino]propyl]-2(S)-pyrrolidinecarboxamide

En opérant de façon analogue à l'exemple 2, au départ du composé obtenu à la préparation XLIV, on obtient le produit attendu sous forme d'un solide beige (Rendement = 47 %).
F = 115°C
[α]²²_{D} = - 9,5° (c = 0,55 ; DMSO)

### Exemple 38

### 1-[[3-[[3-Chloro-2-méthylimidazo[1,2-a]pyridin-8-yl]oxyméthyl]-2,4-dichlorophényl]sulfonyl]-N-[3-[[4-(aminoiminométhyl)benzoyl]amino]propyl]-2(S)-pyrrolidinecarboxamide, bis(méthanesulfonate)

En opérant de façon analogue à l'exemple 30, au départ du composé obtenu selon l'exemple 37, on obtient le produit attendu sous forme d'un solide blanc (Rendement = 90 %).
F = 154°C
[α]²⁷_{D} = - 19° (c = 0,32 ; CH₃OH)

### PREPARATION XLV

### 1-[[3-[[3-Chloro-2-méthylimidazo[1,2-a]pyridin-8-yl]oxyméthyl]-2,4-dichlorophényl]sulfonyl]-2(S)-[[4-(4-cyanobenzoyl)piperazin-1-yl]carbonyl]pyrrolidine

En opérant de façon analogue à la préparation V, au départ des composés obtenus selon les préparations XLII et VIII, on obtient le produit attendu sous forme d'un solide blanc-crème (Rendement = 49 %).
F = 100 ° C
[α]²⁸_{D} = - 11° (c = 0,35 ; CH₃OH)

### Exemple 39

### 1-[[3-[[3-Chloro-2-méthylimidazo[1,2-a]pyridin-8-yl]oxyméthyl]-2,4-dichlorophényl]sulfonyl]-2(S)-[[4-[4-[(amino)(hydroxyimino)méthyl]benzoyl]piperazin-1-yl]carbonyl]pyrrolidine

En opérant de façon analogue à l'exemple 1, au départ du composé obtenu selon la préparation XLV, on obtient le produit attendu sous forme d'un solide blanc (Rendement = 96 %).
F = 168 ° C
[α]²⁸_{D} = - 25° (c = 0,59 ; CH₃OH)

### PREPARATION XLVI

### 1-[[3-[[3-Chloro-2-méthylimidazo[1,2-a]pyridin-8-yl]oxyméthyl]-2,4-dichlorophényl]sulfonyl]-2(S)-[[4-[4-[(acétoxyimino)(amino)méthyl]benzoyl]piperazin-1-yl]carbonyl]pyrrolidine

En opérant de façon analogue à la préparation VI, au départ du composé obtenu selon l'exemple 39, on obtient le produit attendu sous forme d'un solide blanc cassé (Rendement = 88 %).
F = 150 ° C
[α]²⁶_{D} = - 16,5° (c = 0,40 ; CH₃OH)

### Exemple 40

### 1-[[3-[[3-Chloro-2-méthylimidazo[1,2-a]pyridin-8-yl]oxyméthyl]-2,4-dichlorophényl]sulfonyl]-2(S)-[[4-[4-[(aminoiminométhyl)benzoyl]piperazin-1-yl]carbo nyl]pyrrolidine

En opérant de façon analogue à l'exemple 2, au départ du composé obtenu selon la préparation XLVI, on obtient le produit attendu sous forme d'un solide jaune pâle (Rendement = 96 %).
F = 164 ° C
[α]²⁶_{D} = - 14° (c = 0,33 ; CH₃OH)

### Exemple 41

### 1-[[3-[[3-Chloro-2-méthylimidazo[1,2-a]pyridin-8-yl]oxyméthyl]-2,4-dichlorophényl]sulfonyl]-2(S)-[[4-[4-[(aminoiminométhyl)benzoyl]piperazin-1-yl]carbonyl]pyrrolidine, bis(méthanesulfonate)

En opérant de façon analogue à l'exemple 3, (dans le cas présent, on utilise le dichlorométhane pour dissoudre le composé de départ), au départ du composé obtenu selon l'exemple 40, on obtient le produit attendu sous forme d'un solide fin blanc (Rendement = 83 %).
F = 192 ° C
[α]²⁷_{D} = + 21° (c = 0,42 ; CH₃OH)

### PREPARATION XLVII

### N-[[3-[(2-Amino-pyridin-3-yl)oxyméthyl]-2,4-dichlorophényl]sulfonyl]-(L)-proline, méthyl ester

On prépare une solution de 4 g (36,4.10⁻³ mole) de 2-amino-3-hydroxy-pyridine dans 200 ml de diméthylformamide et on ajoute, à température ambiante, 1,09 g (36,4.10⁻³ mole) d'hydrure de sodium. Après 30 mn sous agitation, on ajoute 15,7 g (36,4.10⁻³ mole) d'ester méthylique de la N-[(3-bromométhyl-2,4-dichlorophényl)sulfonyl]-(L)-proline, et on agite le mélange pendant 2 heures à température ambiante. Le milieu réactionnel est ensuite versé sur 250 ml d'eau glacée. Le produit est séparé par filtration et repris en solution dans de l'acétate d'éthyle. La solution est lavée à l'eau puis séchée sur sulfate de sodium et concentrée sous pression réduite. Le produit brut obtenu est purifié par chromatographie sur gel de silice en éluant avec un mélange dichlorométhane/méthanol (97/3 ; v/v). On obtient ainsi 12,4 g du composé attendu sous forme de cristaux jaune-orangé (Rendement = 74,5 %).
F = 68 °C
[α]²⁵_{D} = - 12,8° (c = 1,05 ; CH₃OH)

### PREPARATION XLVIII

### N-[[3-[[3-Acétyl-2-méthylimidazo[1,2-a]pyridin-8-yl]oxyméthyl]-2,4-dichlorophényl]sulfonyl]-proline, méthyl ester

On prépare une solution de 10,1 g (22.10⁻³ mole) du composé obtenu selon la préparation XLVII, dans 160 ml d'éthanol. On ajoute sous agitation 6 ml (50.10⁻³ mole) de 3-chloro-2,4-pentanedione et on agite à reflux du solvant pendant 15 heures. Le mélange réactionnel est ensuite concentré sous pression réduite et le produit brut obtenu est purifié par chromatographie sur gel de silice en éluant avec un mélange méthylcyclohexane/acétate d'éthyle (1/1 ; v/v). On obtient ainsi 2,67 g du produit attendu sous forme de cristaux beiges (Rendement = 22,5 %).
F = 178 °C
[α]²⁵_{D} = - 8,6° (c = 1,05 ; DMSO)

### PREPARATION IL

### N-[[3-[[3-Acétyl-2-méthylimidazo[1,2-a]pyridin-8-yl]oxyméthyl]-2,4-dichlorophényl]sulfonyl]-proline

En opérant de façon analogue à la préparation II, au départ du composé obtenu selon la préparation XLVIII, on obtient le produit attendu sous forme de cristaux blanc-crème (Rendement = 91 %).
F = 162-164 °C
[α]²⁵_{D} = - 8,3° (c = 1,01; CH₃OH)

### PREPARATION L

### 1-[[3-[[3-Acétyl-2-méthylimidazo[1,2-a]pyridin-8-yl]oxyméthyl]-2,4-dichlorophényl]sulfonyl]-N-[3-[(4-cyanobenzoyl)amino]propyl]-2(S)-pyrrolidine carboxamide

En opérant de façon analogue à la préparation V, au départ du composé obtenu à la préparation IL, on obtient le produit attendu sous forme de cristaux blanc-cassé (Rendement = 94 %).
F = 100-102°C
[α]²⁰_{D} = - 30,6° (c = 1,02 ; CHCl₃)

### Exemple 42

### 1-[[3-[[3-Acétyl-2-méthylimidazo[1,2-a]pyridin-8-yl]oxyméthyl]-2,4-dichlorophényl]sulfonyl]-N-[3-[[4-[(amino)(hydroxyimino)méthyl]benzoyl]amino]-propyl]-2(S)-pyrrolidinecarboxamide

En opérant de façon analogue à l'exemple 1, au départ du composé obtenu à la préparation L, on obtient le produit attendu sous forme de cristaux blancs (Rendement = 88 %).
F = 164-166 °C
[α]²⁵_{D} = - 22° (c = 1,00; CH₃OH)

### PREPARATION LI

### 1-[[3-[[3-Acétyl-2-méthylimidazo[1,2-a]pyridin-8-yl]oxyméthyl]-2,4-dichlorophényl]sulfonyl]-N-[3-[[4-[(acétoxyimino)(amino)méthyl]benzoyl]amino]-propyl]-2(S)-pyrrolidinecarboxamide

En opérant de façon analogue à la préparation VI, au départ du composé obtenu selon l'exemple 42, on obtient le produit attendu sous forme de cristaux blancs (Rendement = 97 %).
F = 115-117°C
[α]²⁴_{D} = - 6,4° (c = 1,06 ; DMSO)

### Exemple 43

### 1-[[3-[[3-Acétyl-2-méthylimidazo[1,2-a]pyridin-8-yl]oxyméthyl]-2,4-dichlorophényl]sulfonyl]-N-[3-[[4-(aminoiminométhyl)benzoyl]amino]propyl]-2(S)-pyrrolidinecarboxamide

En opérant de façon analogue à l'exemple 2, au départ du composé obtenu à la préparation LI, on obtient le produit attendu sous forme de cristaux blancs (Rendement = 69 %).
F = 158-160°C
[α]²¹_{D} = - 9,5° (c = 1,01 ; DMSO)

### Exemple 44

### 1-[[3-[[3-Acétyl-2-méthylimidazo[1,2-a]pyridin-8-yl]oxyméthyl]-2,4-dichlorophényl]sulfonyl]-N-[3-[[4-(aminoiminométhyl)benzoyl]amino]propyl]-2(S)-pyrrolidinecarboxamide, bis(méthanesulfonate)

En opérant de façon analogue à l'exemple 3, au départ du composé obtenu selon l'exemple 43, on obtient le produit attendu sous forme de cristaux blancs (Rendement de 84 %).
F = 170-172°C
[α]²²_{D} = - 7,5° (c = 1,02 ; DMSO)

L'activité des produits selon l'invention a été évaluée en fonction de leur aptitude à se lier aux récepteurs de la bradykinine. En effet, les kinines, dont le principal représentant est la bradykinine, forment un groupe de petits peptides qui contribuent de façon importante à la réponse inflammatoire et apparaissent de ce fait impliqués dans la pathophysiologie des maladies inflammatoires. De plus, la bradykinine est un des agents algésiants parmi les plus puissants connus. Les kinines activent deux types de récepteurs appelés respectivement B₁ et B₂. Le récepteur B₂ appartient à la grande famille des récepteurs à sept domaines transmembranaires couplés aux G-protéines. Nous décrivons dans la présente invention des composés se liant au récepteur B₂ et bloquant de ce fait la fixation de la bradykinine.

Le test pharmacologique utilisé est le suivant : des segments d'iléon de cobayes mâles [de souche Dunkin-Hartley (Iffa Credo, l'Arbresle, France)] sont isolés et broyés dans le tampon TES suivant : TES 25mM, 1,10-phénanthroline 1mM (pH 6.8), bacitracine 140 µg/ml, BSA 1g/l. Les membranes sont ensuite isolées par centrifugation (18000 tours par minute ; 20 min ; 4°C). Les études de liaison sont effectuées dans ce tampon TES en utilisant la [³H]-bradykinine (120 pM), 50 µg de protéine membranaire par essai (volume final 500 µl) avec un temps d'équilibre de 90 min à 20°C. On détermine ensuite le taux (en pourcentage) d'inhibition de la fixation de [³H]-bradykinine en présence de l'un des composés selon l'invention à tester à une concentration de 10⁻⁶M.

Les résultats obtenus (notés "activité") lors de ces essais sont consignés dans le tableau I ci-après en regard des exemples figurant dans la description. Dans ce tableau, la signification des restes hétérocycles Q est donnée dans les notes et la nature des sels est indiquée par les abréviations Chl quand il s'agit d'un sel avec l'acide chlorhydrique et Ms quand il s'agit d'un sel avec l'acide méthanesulfonique.

Les composés selon l'invention ont un effet antagoniste vis-à-vis du récepteur B₂ de la bradykinine (ils inhibent l'activation des récepteurs B₂ induite par la bradykinine). Cette propriété a été démontrée expérimentalement au moyen de tests pharmacologiques décrits dans la publication de D. PRUNEAU et coll. (British. Journal of Pharmacology, oct 1995, vol 116 (n° 3) p 2106-2112) et réalisés sur l'iléon de cobaye. Les résultats expérimentaux, évalués par le pK_{B} décrit dans la publication ci-dessus, sont reportés dans le tableau récapitulatif.

Les composés de la présente invention, qui inhibent la liaison de la [³H]-bradykinine au récepteur B₂ de cobaye (voir tableau I), se lient également au récepteur B₂ humain cloné et transfecté de façon stable dans des cellules CHO (en anglais : "Chinese Hamster Ovary Cells"). Ainsi dans ce test, certains composés inhibent à la concentration de 10 µM d'au moins 95 % la fixation de la [³H]-bradykinine au récepteur B₂.

Les composés de la présente invention peuvent être utiles dans le traitement des algies, et en particulier dans le traitement de nombreuses pathologies impliquant la bradykinine ou ses homologues. Parmi ces pathologies, on inclut les chocs septiques et hémorragiques, les réactions anaphylactiques, l'arthrose, la polyarthrite rhumatoïde, les rhinites, l'asthme, les maladies inflammatoires du tractus gastro-intestinal (par ex. colites, rectites, maladie de Crohn), la pancréatite, certains carcinomes, l'angiooedème héréditaire, la migraine, l'encéphalomyélite, la méningite, les accidents vasculaires cérébraux (notamment ceux provoqués par un choc traumatique cérébral), certains désordres neurologiques, les états inflammatoires vasculaires (par exemple : athérosclérose et artérite des membres inférieurs), les états douloureux (par exemple les céphalalgies, les douleurs dentaires, les douleurs menstruelles), les contractions utérines prématurées, la cystite et les brûlures. Les composés selon l'invention peuvent également être utiles pour la potentialisation d'agents antiviraux.

Les composés de la présente invention, qui peuvent être utilisés sous forme de base libre ou de leurs sels d'addition non-toxiques, en association avec un excipient physiologiquement acceptable, sont en général prescrits en thérapeutique humaine à des doses d'environ 1 à 1000 mg/jour, sous une forme administrable par voie orale, par injection intraveineuse, intramusculaire ou sous-cutanée, par voie transdermique, par le moyen d'aérosols ou par le moyen de suppositoires.

Ces composés sont également administrables par voie topique, par exemple sous forme de gel ou de pommade.

Les composés de la présente invention trouvent également leur utilité, en tant que réactifs pharmacologiques, notamment pour l'étude des interactions hormone-récepteur. L'utilisation en tant que réactif pharmacologique peut faire appel à un dérivé radiomarqué de l'un des composés selon l'invention (par exemple avec du tritium [³H] ou du soufre [³⁵S]), dans le but d'obtenir un radio-ligand destiné à des études conformationnelles du récepteur B₂ de la bradykinine, ou des tests de fixation à ce type de récepteur, par exemple pour l'évaluation de nouveaux composés susceptibles de présenter une affinité pour le récepteur B₂ de la bradykinine.

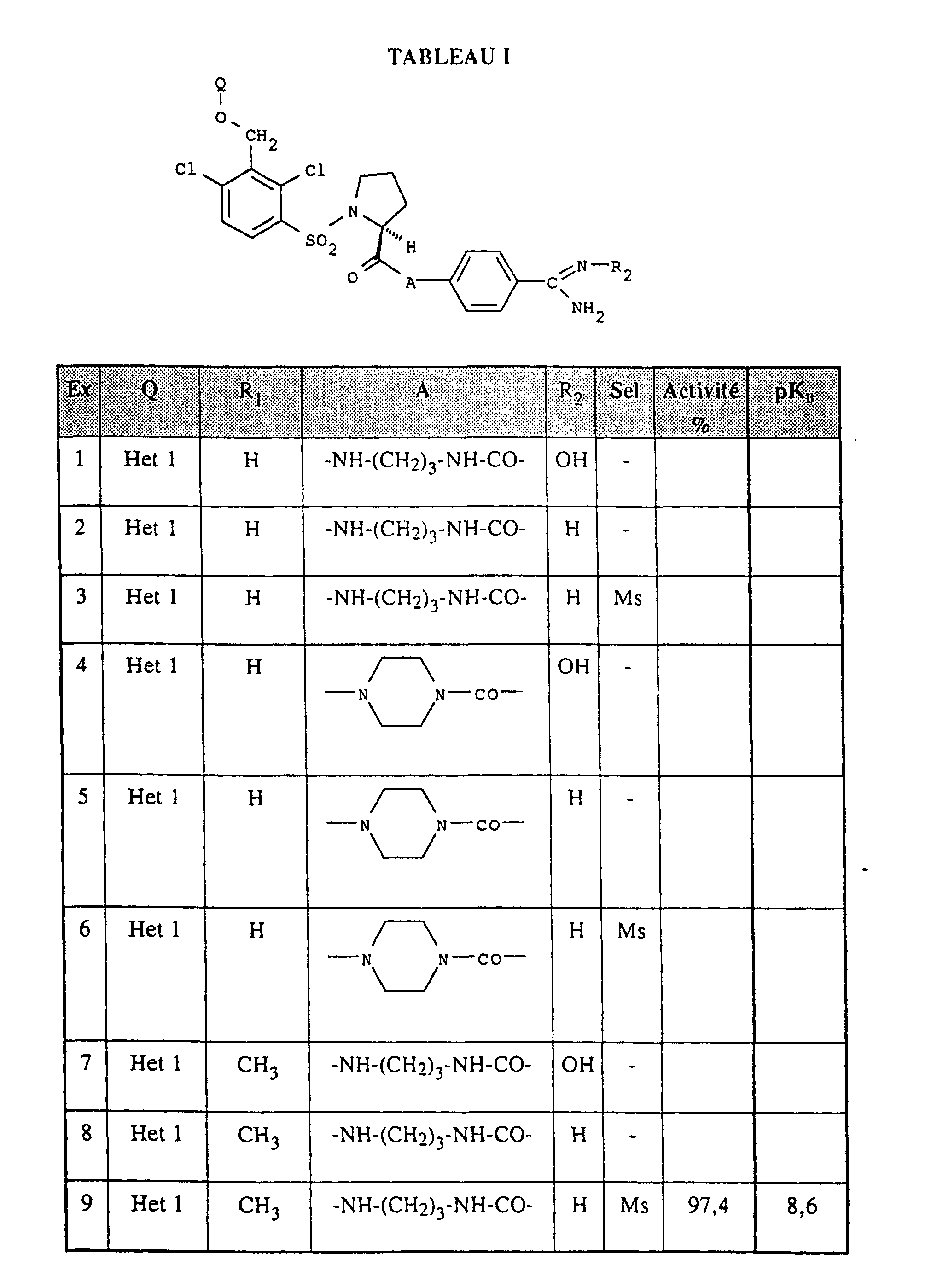

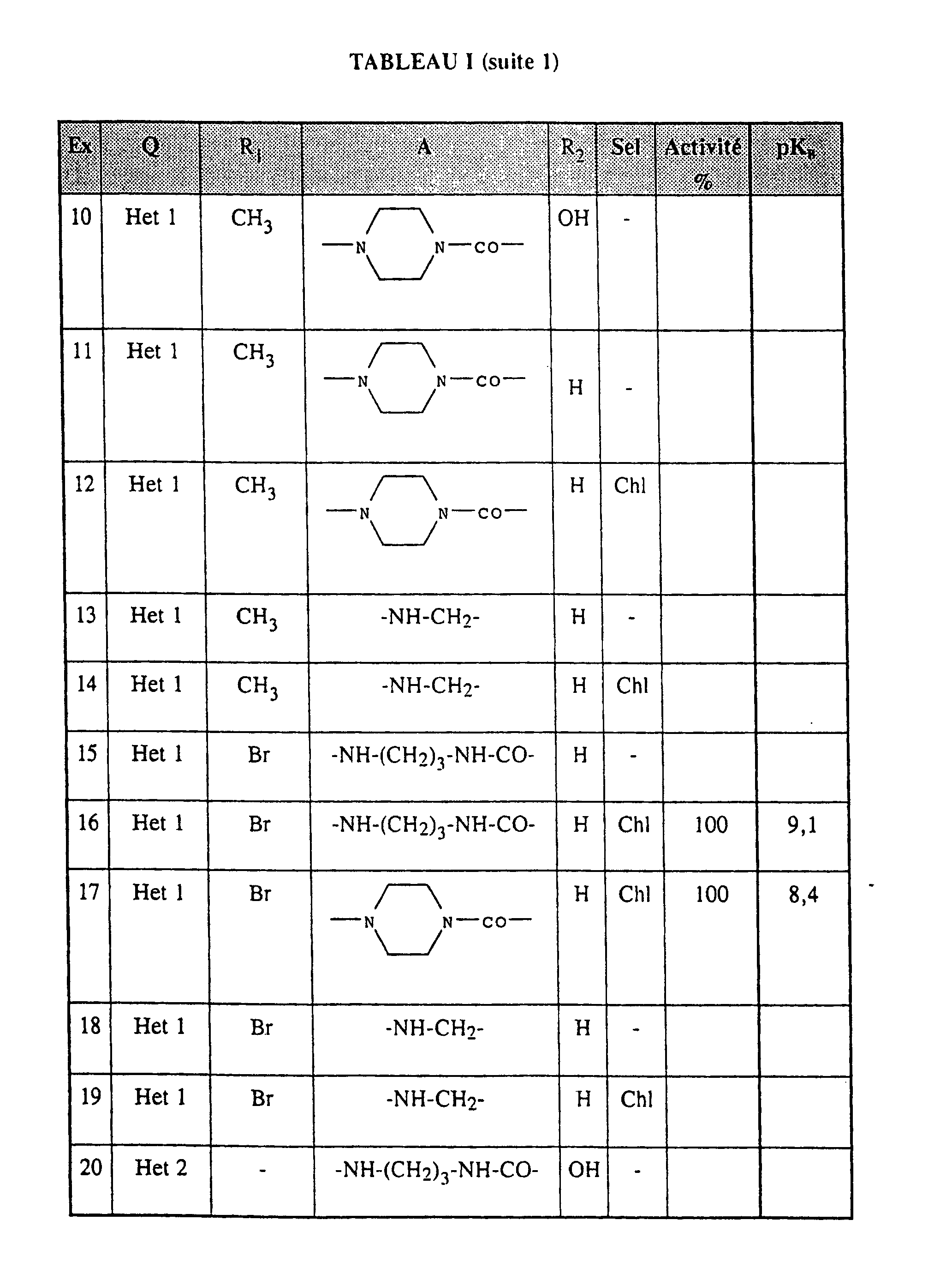

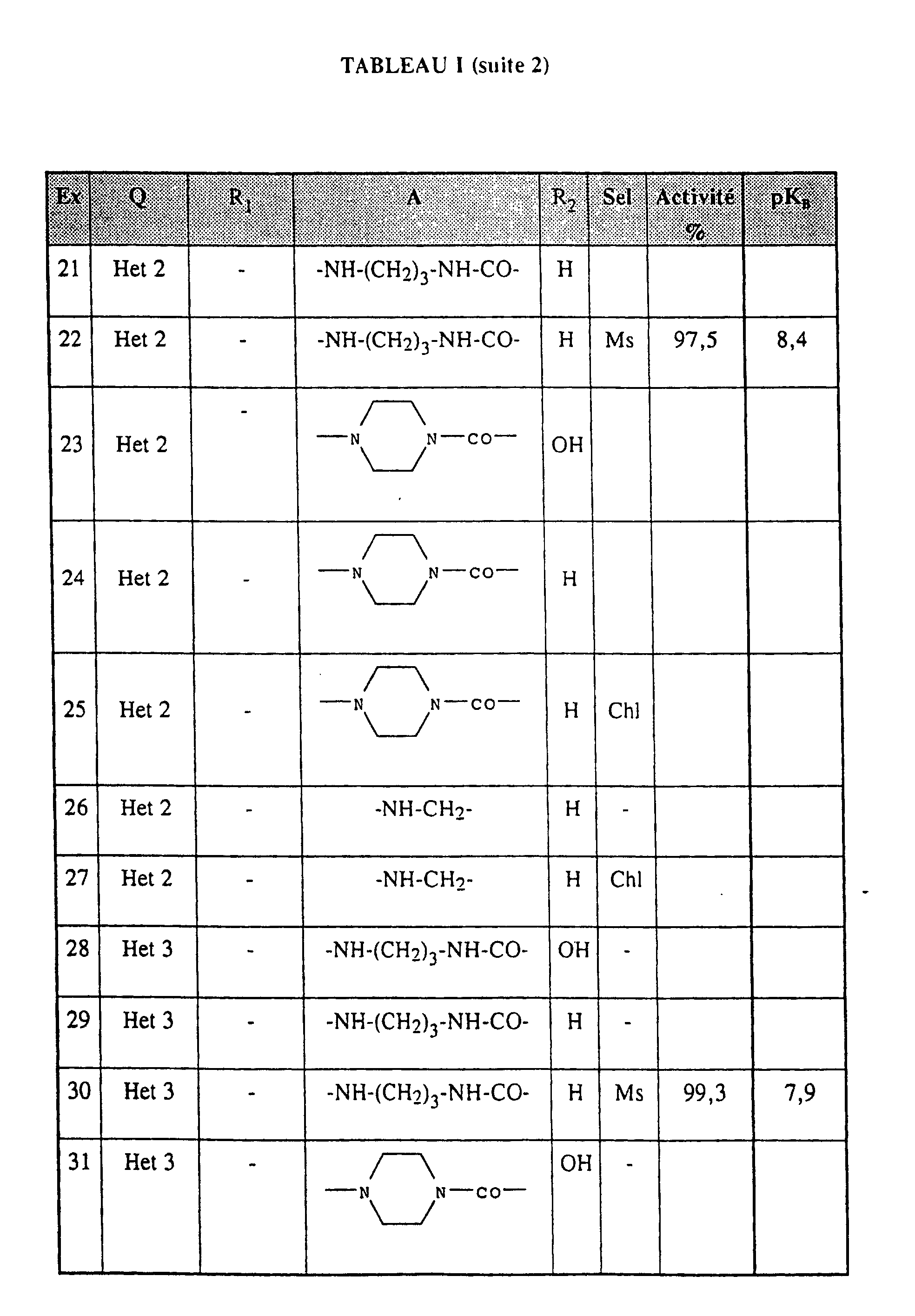

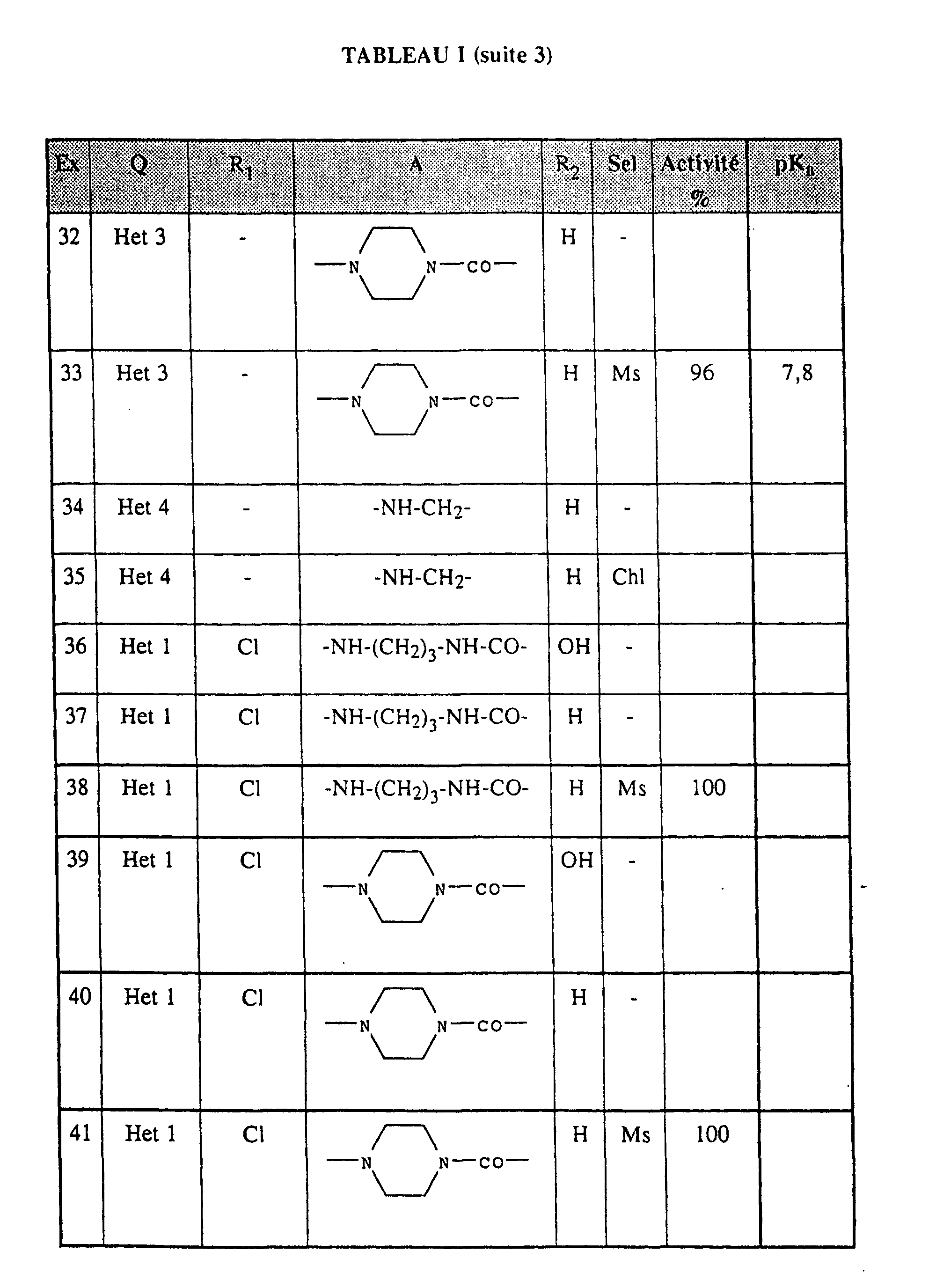

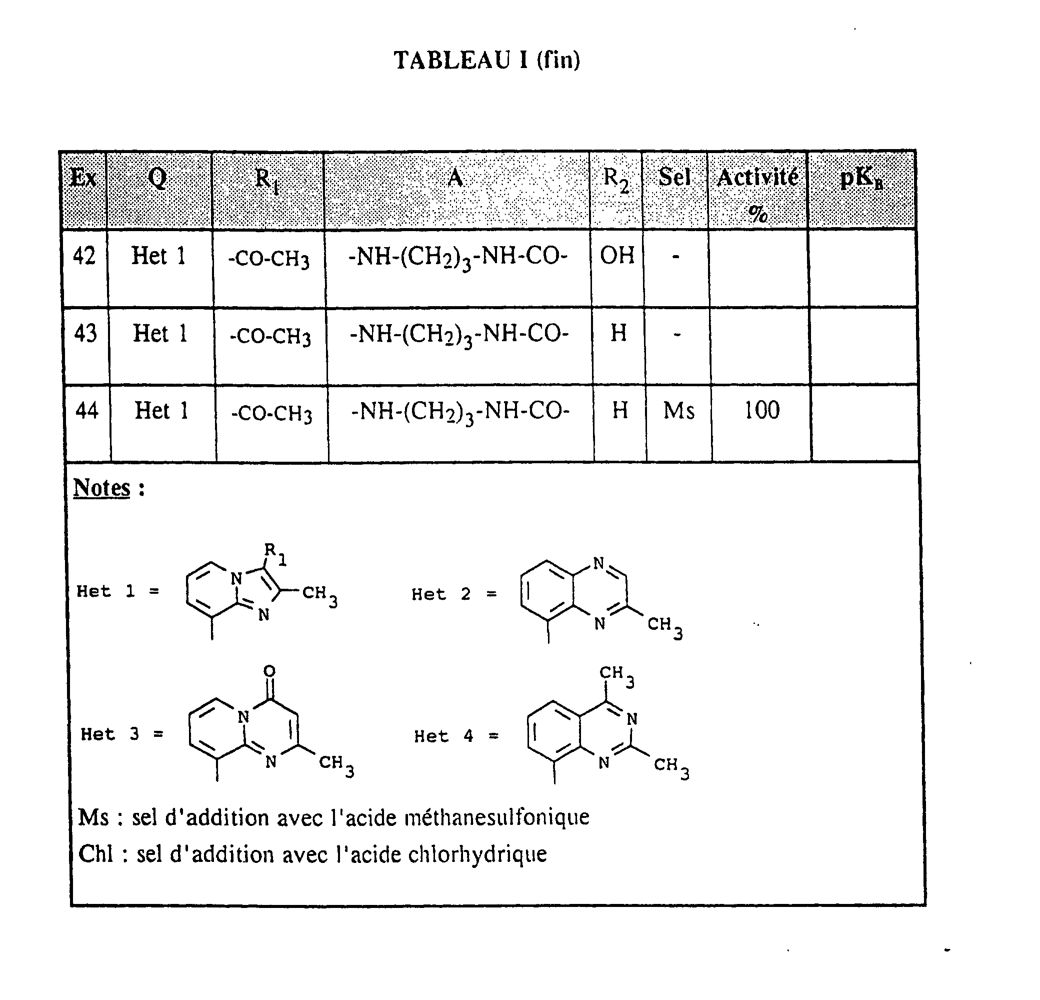

## Revendications

1. Composé de N-(benzènesulfonyl)-L-proline, **caractérisé en ce qu'**il est choisi parmi l'ensemble constitué par :
(i) les composés de formule l : 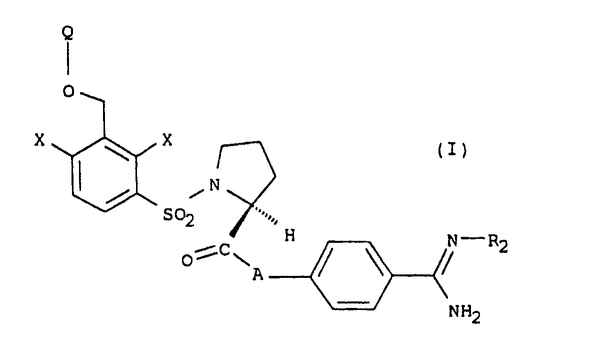 dans laquelle :
X représente un atome d'halogène,
A représente un groupe divalent 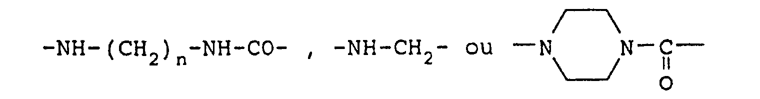
Q représente un groupe 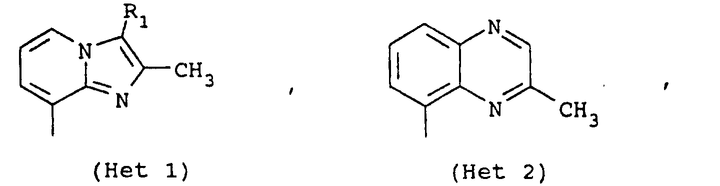 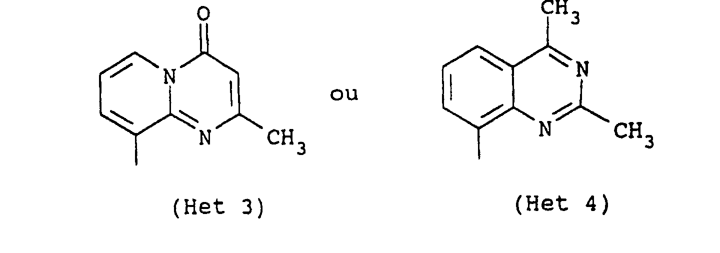
R₁ représente un atome d'hydrogène, un atome d'halogène, un groupe alkyle en C₁-C₃ à chaîne hydrocarbonée linéaire ou ramifiée ou un groupe 1-oxoalkyle en C₁-C₅,
R₂ représente un atome d'hydrogène ou un groupe OH,
n représente 2, 3, ou 4, et
(ii) leurs sels d'addition.

2. Composé selon la revendication 1, **caractérisé en ce que** X est Cl.

3. Procédé de préparation d'un composé de formule I, **caractérisé en ce qu'**il comprend
selon une première variante A, les étapes consistant à :
(1) faire réagir un composé hétérocyclique hydroxylé de formule :
Q-O-Met
dans laquelle
Met représente un métal alcalin, notamment Na ou K,
Q représente un groupe hétérocyclique choisi parmi les structures Het 1,
Het 2, Het 3 et Het 4 : 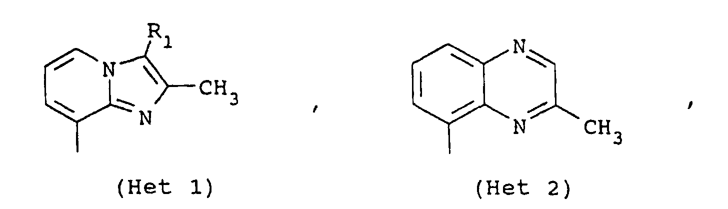 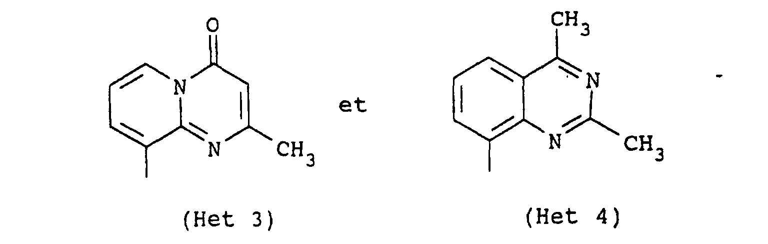
R₁ représentant un atome d'hydrogène ou un groupe alkyle en C₁-C₃, avec un composé de formule II : 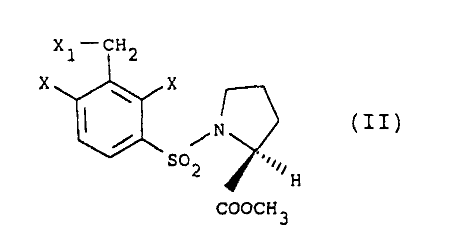 dans laquelle X représente un atome d'halogène et X₁ représente un atome d'halogène, de préférence Br,
dans un solvant anhydre, à une température comprise entre 0 et 50°C et pendant 0.5 à 10 heures, pour obtenir un composé de formule III : 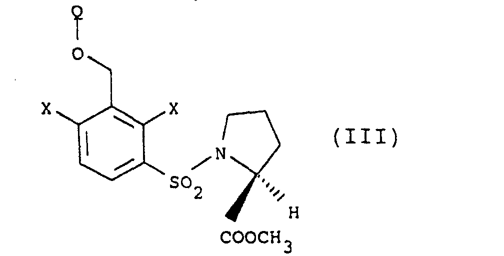 dans laquelle Q, X et R₁ conservent la même signification que précédemment ;
(2) si nécessaire, lorsque dans le composé de formule III Q représente le groupe Het 1 où R₁ est un atome d'hydrogène : 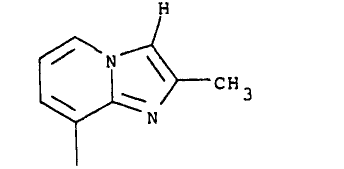 faire réagir ledit composé de formule III avec un agent halogénant dans un solvant, à une température comprise entre environ 0 et 50°C et pendant 0,5 à 20 heures, pour obtenir un composé de formule III' : 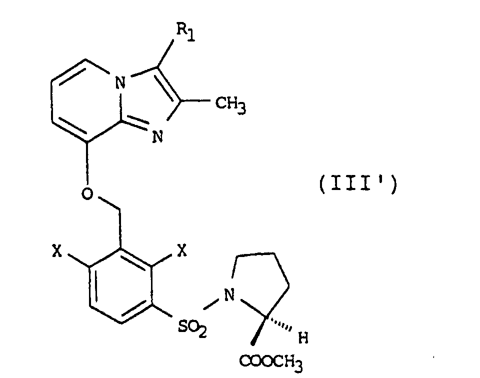 dans laquelle
R₁ représente un atome d'halogène, de préférence Br ou Cl ;
(3) hydrolyser la fonction ester du composé de formule III ou III' obtenu selon l'une des étapes (1) ou (2) ci-dessus, à une température de l'ordre de 20 à 60°C, pendant 1 à 5 heures, pour obtenir un composé de formule IV : 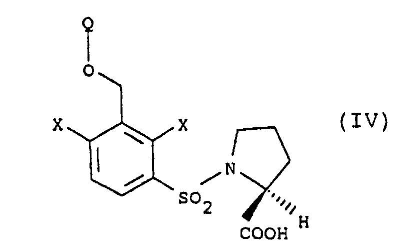 dans laquelle Q et X conservent la même signification que ci-dessus et R₁ représente un atome d'hydrogène, un atome d'halogène ou un groupe alkyle en C₁-C₃ ;
(4) faire réagir le composé de formule IV, ainsi obtenu, avec le sel d'une amine de formule : 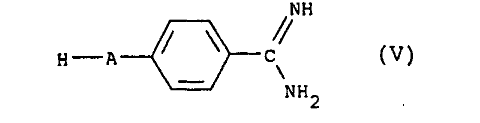 dans laquelle A représente un groupe 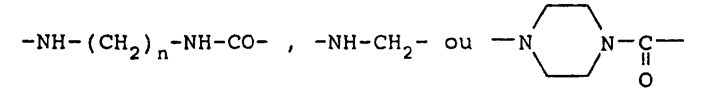 où n représente 2, 3 ou 4, dans un solvant en présence d'activateurs tels que notamment le 1-hydroxy-7-aza-benzotriazole (HOAT) et le chlorhydrate de 1-[3-(diméthylaminopropyl)-3-éthyl]-carbodiimide (EDCI), à une température voisine de la température ambiante (10-35°C), et pendant 2 à 50 heures, pour obtenir un composé de formule I où R₂ est H: 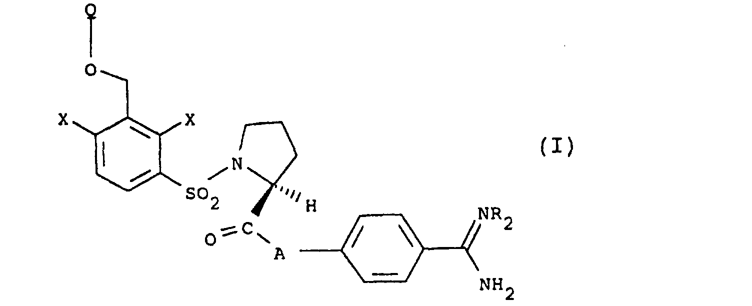 dans laquelle A, Q, X et R₁ conservent la même signification que précédemment et R₂ est H ; et,
(5) si nécessaire, faire réagir le composé de formule I ainsi obtenu, avec un acide pour obtenir le sel d'addition d'acide correspondant ;
selon une seconde variante B, les étapes consistant à :
(1) faire réagir le composé acide de formule IV obtenu, par exemple, à l'étape (3) de la variante A dans lequel R₁ est un atome d'hydrogène, un atome de chlore, un groupe alkyle en C₁-C₃ à chaîne hydrocarbonée linéaire ou ramifiée ou un groupe 1-oxoalkyle en C₁-C₅,
avec un composé de formule VI : 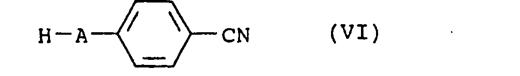 dans laquelle A représente un groupe 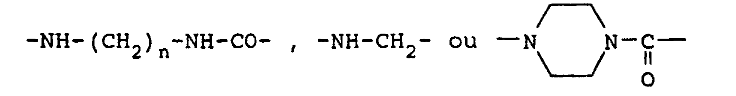 où n représente 2, 3 ou 4,
dans des conditions analogues à celles de l'étape (4) de la variante A ci-dessus, pour obtenir un composé de formule VII : 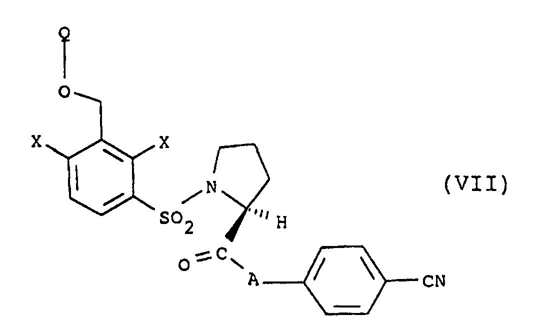 dans laquelle Q, R₁, X et A conservent la même signification que dans les produits de départ ;
(2) faire réagir le composé de formule VII, ainsi obtenu, avec de l'hydroxylamine, dans un solvant à température ambiante (15-25°C), pendant 1 à 12 heures, pour obtenir un composé de formule VIII : 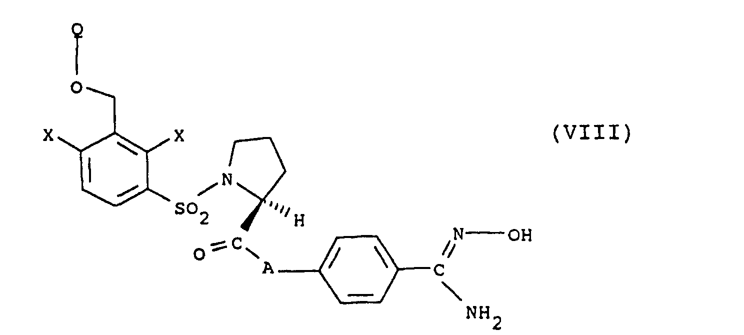 dans laquelle Q, R₁, X et A conservent la même signification que précédemment ;
(3) acétyler le composé de formule VIII, ainsi obtenu, à une température proche de la température ambiante pendant 1 à 8 heures, pour obtenir le composé de formule IX : 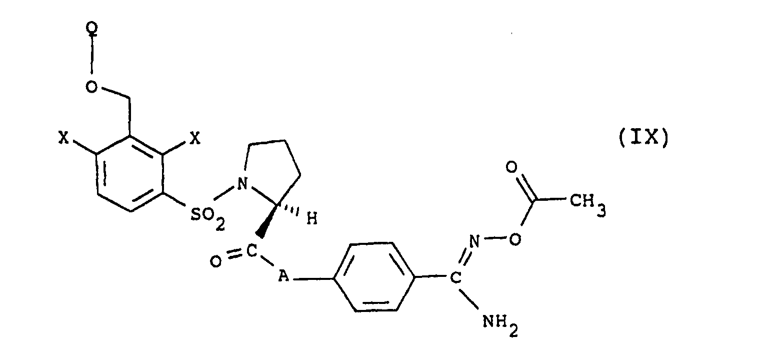 dans laquelle Q, R₁, X et A conservent la même signification que ci-dessus ;
(4) effectuer une réduction par hydrogénation catalytique du composé de formule IX, ainsi obtenu, à une température proche de la température ambiante, sous une pression d'hydrogène comprise entre 10⁵ et 10⁶ Pascals, pour obtenir le composé de formule I dans laquelle Q, R₁, X et A conservent la même signification que ci-dessus et R₂ est H ;
selon une troisième variante C, les étapes consistant à :
(1) faire réagir le composé de formule V obtenu conformément à l'étape (3) de la variante A avec une amine de formule 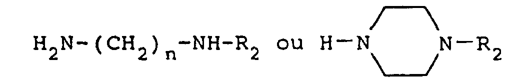 où n représente 2, 3 ou 4, et R₂ représente un groupe aminoprotecteur,
notamment le groupe 1,1-diméthyléthoxycarbonyle,
dans des conditions opératoires analogues à celles de l'étape (4) de la variante A, pour obtenir un composé de formule X : 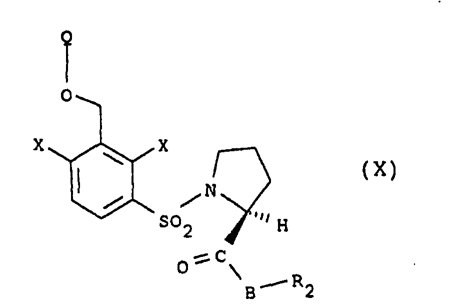 dans laquelle B représente 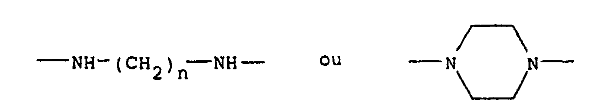 et Q, R₁ et X conservent la même signification que précédemment ;
(2) effectuer la déprotection de la fonction amine du composé de formule X, ainsi obtenu, de façon à remplacer le groupe R₂ par un atome d'hydrogène et obtenir le composé de formule XI : 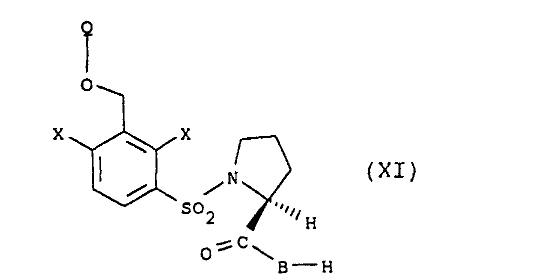 dans laquelle B, Q, R₁ et X conservent la même signification que ci-dessus ; et,
(3) faire réagir le composé de formule XI, ainsi obtenu, avec l'acide 4-(amino-iminométhyl)benzoïque, dans des conditions analogues à celles de l'étape (4) de la variante A ci-dessus, pour obtenir le composé de formule I où R₂ est H : 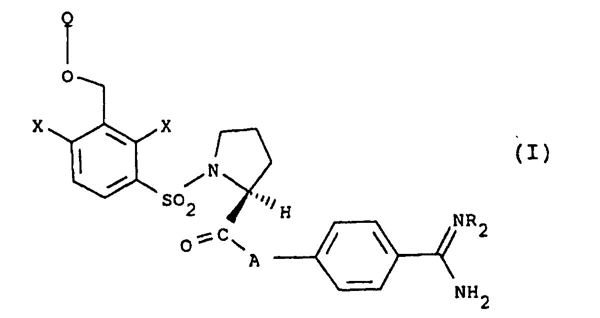 dans laquelle
Q, R₁ et X conservent la même signification que précédemment, R₂ est H et
A représente un groupe : 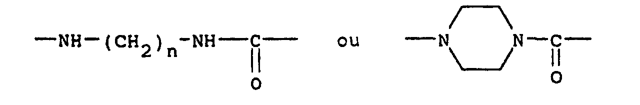
et selon une quatrième variante D, les étapes consistant à :
(1) faire réagir l'acide de formule : 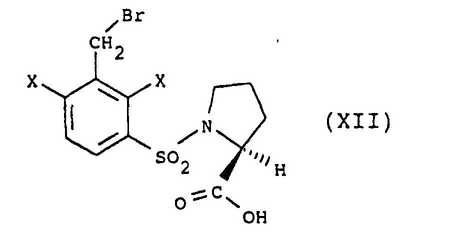 dans laquelle X représente un halogène,
avec un composé de formule 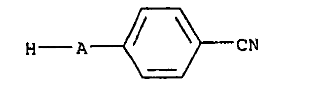 dans laquelle A représente un groupe : 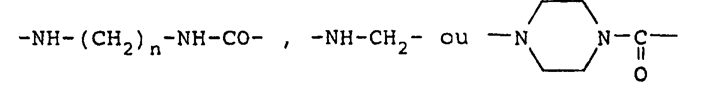 où n représente 2, 3 ou 4,
dans des conditions analogues à celles de l'étape (4) de la variante A ci-dessus, et obtenir le composé de formule XIII : 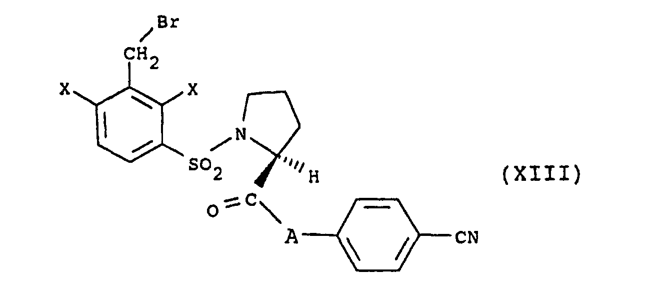 dans laquelle A et X conservent la même signification que ci-dessus ;
(2) faire réagir le composé de formule XIII, ainsi obtenu, avec un dérivé hétérocyclique hydroxylé de structure générale Q-OH, Q étant choisi parmi les structures : 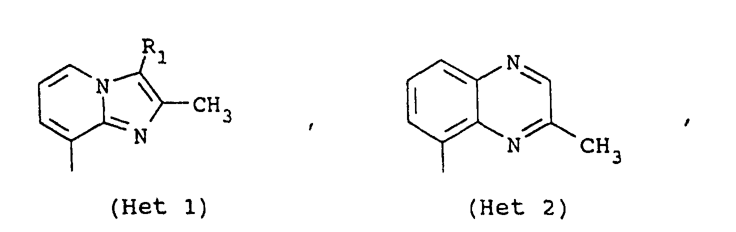 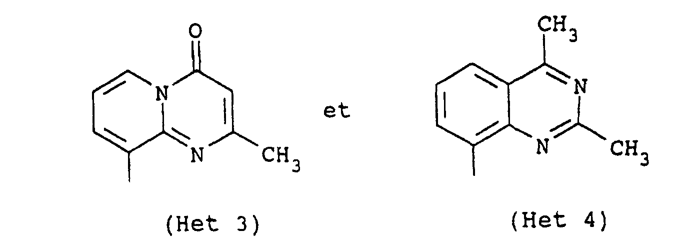 où R₁ représente un atome d'hydrogène, un atome d'halogène, un groupe alkyle en C₁-C₃ ou un groupe 1-oxoalkyle en C₁-C₅,
dans des conditions analogues à celles de l'étape (1) de la variante A ci-dessus, pour obtenir le composé de formule XIV : 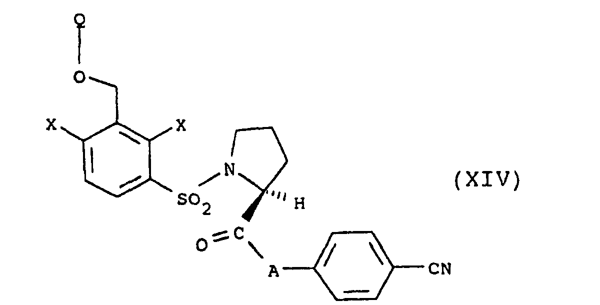 dans laquelle Q, R₁, X et A conservent la même signification que dans les produits de départ ; et,
(3) effectuer ensuite sur le composé de formule XIV, ainsi obtenu, une série de réactions analogues à celles précédemment décrites aux étapes (2), (3) et (4) de la variante B ci-dessus, pour obtenir le composé de formule I où Q, X, R₁ et A conservent la même signification que dans les composés de départ.

4. Composition thérapeutique **caractérisée en ce qu'**elle renferme, en association avec un excipient physiologiquement acceptable, au moins un composé choisi parmi l'ensemble constitué par les composés de formule I et leurs sels d'addition non-toxiques selon la revendication 1.

5. Utilisation d'une substance antagoniste d'un récepteur de la bradykinine et des hormones analogues, ladite utilisation étant **caractérisée en ce que** l'on fait appel à une substance antagoniste du récepteur B₂ de la bradykinine et choisie parmi l'ensemble constitué par les composés de formule I et leurs sels d'addition non toxiques selon la revendication 1, pour l'obtention d'un médicament destiné à une utilisation en thérapeutique vis-à-vis d'états pathologiques impliquant la bradykinine ou ses homologues.

6. Utilisation selon la revendication 5, d'une substance choisie parmi l'ensemble constitué par les composés de formule I et leurs sels d'addition non toxiques selon la revendication 1, pour l'obtention d'un médicament destiné à une utilisation en thérapeutique pour le traitement d'états douloureux.

7. Utilisation selon la revendication 5, d'une substance choisie parmi l'ensemble constitué par les composés de formule I et leurs sels d'addition non toxiques selon la revendication 1, pour l'obtention d'un médicament destiné à une utilisation en thérapeutique pour le traitement d'états inflammatoires.

8. Utilisation d'un composé de formule I ou l'un de ses sels d'addition selon la revendication 1, en tant que réactif biologique destiné à permettre d'évaluer l'affinité d'un composé chimique pour le récepteur B₂ de la bradykinine.

## Patentansprüche

1. N-(Benzolsulfonyl)-L-prolin-Verbindung, **dadurch gekennzeichnet, dass** sie ausgewählt ist aus:
(i) den Verbindungen der Formel I: 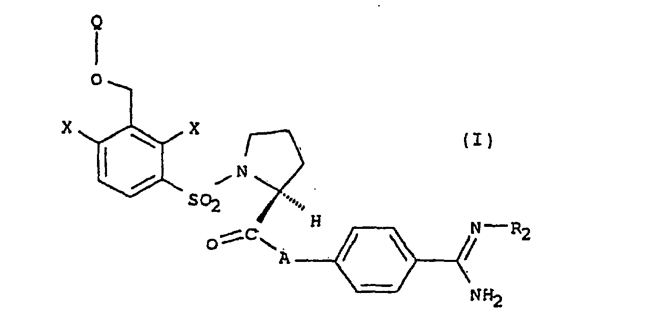 wobei:
X ein Halogenatom darstellt,
A eine zweiwertige Gruppe 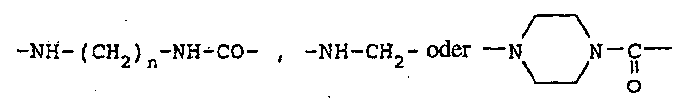 darstellt,
Q eine Gruppe 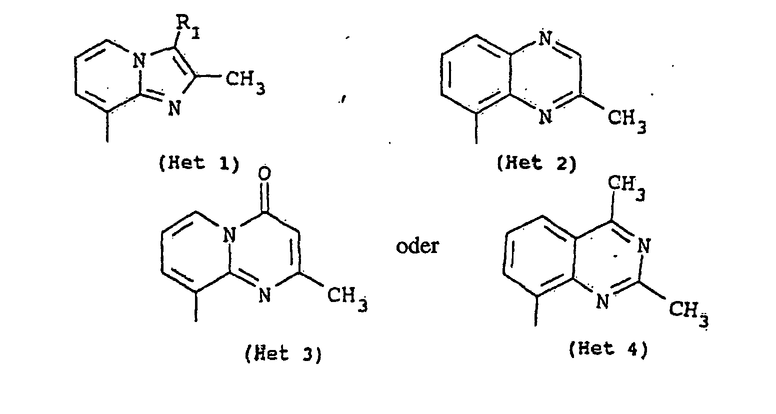 darstellt,
R₁ ein Wasserstoffatom, ein Halogenatom, einen C₁-C₃-Alkylrest mit einer linearen oder verzweigten Kohlenwasserstoffkette oder einen 1-Oxo-C₁-C₅alkylrest darstellt,
R₂ ein Wasserstoffatom oder eine OH-Gruppe darstellt,
n 2, 3 oder 4 ist und
(ii) ihren Additionssalzen.

2. Verbindung gemäß Anspruch 1, **dadurch gekennzeichnet, dass** X ein Chloratom ist.

3. Verfahren zur Herstellung einer Verbindung der Formel I, **dadurch gekennzeichnet, dass** es gemäß einer ersten Variante A die Schritte umfasst:
(1) Umsetzen einer hydroxylierten heterocyclischen Verbindung der Formel:
Q-O-Met
wobei
Met ein Alkalimetall, insbesondere Na oder K, darstellt,
Q eine heterocyclische Gruppe, ausgewählt aus den Strukturen Het 1, Het 2, Het 3 und Het 4, darstellt: 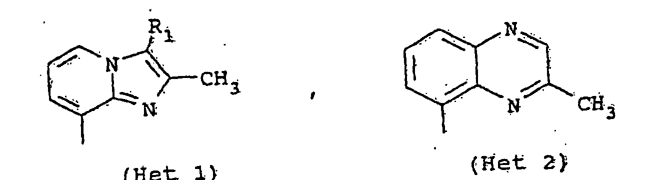 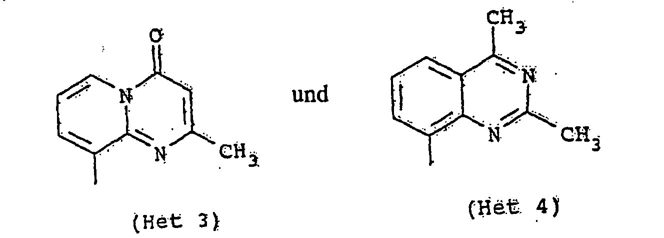 R₁ ein Wasserstoffatom oder ein C₁-C₃-Alkylrest darstellt,
mit einer Verbindung der Formel II: 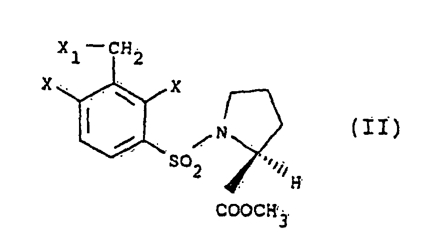 wobei X ein Halogenatom darstellt und X₁ ein Halogenatom, vorzugsweise ein Bromatom, darstellt,
in einem wasserfreien Lösungsmittel bei einer Temperatur zwischen 0 und 50 °C für 0,5 bis 10 Stunden, um eine Verbindung der Formel III zu erhalten: 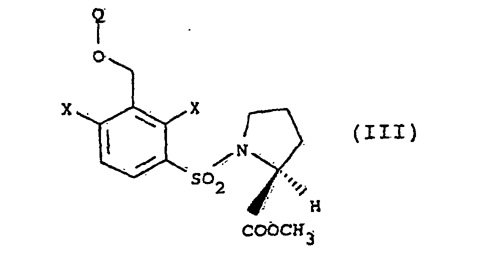 wobei Q, X und R₁ die vorstehend angegebene Bedeutung haben;
(2) gegebenenfalls, falls Q in der Verbindung der Formel III die Gruppe Het 1 darstellt, wobei R₁ ein Wasserstoffatom ist: 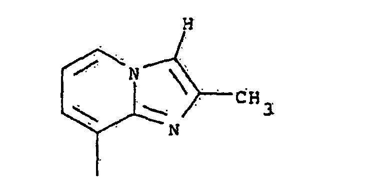 Umsetzen der Verbindung der Formel III mit einem Halogenierungsmittel in einem Lösungsmittel bei einer Temperatur zwischen etwa 0 und 50 °C, für 0,5 bis 20 Stunden, um eine Verbindung der Formel III' zu erhalten: 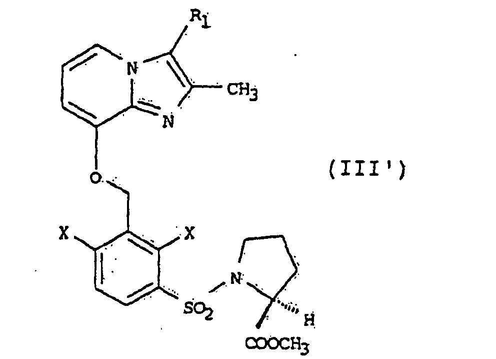 wobei
R₁ ein Halogenatom, vorzugsweise ein Bromatom oder ein Chloratom, darstellt;
(3) Hydrolysieren der Esterfunktion der gemäß einem der vorstehend genannten Schritte (1) oder (2) erhaltenen Verbindung der Formel III oder III' bei einer
Temperatur der Größenordnung von 20 bis 60 °C für 1 bis 5 Stunden, um eine Verbindung der Formel IV zu erhalten: 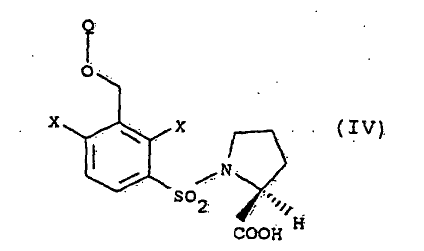 wobei Q und X die vorstehend angegebene Bedeutung haben und R₁ ein Wasserstoffatom, ein Halogenatom oder einen C₁-C₃-Alkylrest darstellt;
(4) Umsetzen der so erhaltenen Verbindung der Formel IV mit dem Salz eines Amins der Formel: 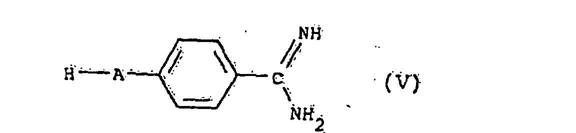 wobei A eine Gruppe 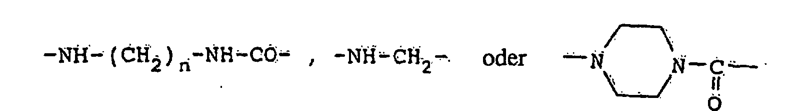 darstellt,
wobei n 2, 3 oder 4 ist,
in einem Lösungsmittel, in Gegenwart von Aktivatoren, wie insbesondere 1-Hydroxy-7-aza-benzotriazol (HOAT) und dem Hydrochlorid von 1-[3-(Dimethylaminopropyl)-3-ethyl]carbodiimid (EDCl), bei einer Temperatur nahe der Raumtemperatur (10-35°C) für 2 bis 50 Stunden, um eine Verbindung der Formel I zu erhalten, wobei R₂ ein Wasserstoffatom ist: 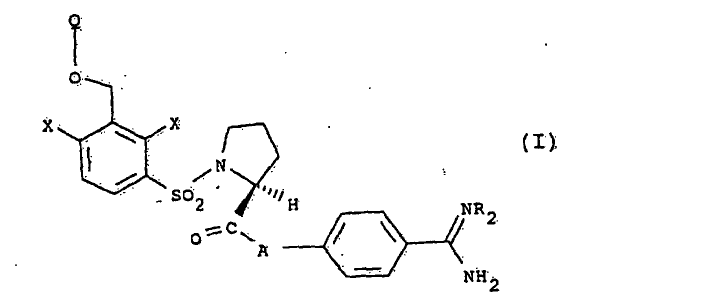 wobei A, Q, X und R₁ die vorstehend angegebene Bedeutung haben und R₂ ein Wasserstoffatom ist; und
(5) gegebenenfalls Umsetzen der so erhaltenen Verbindung der Formel I mit einer Säure, um das Additionssalz der entsprechenden Säure zu erhalten;
dass es gemäß einer zweiten Variante B die Schritte umfasst:
(1) Umsetzen der zum Beispiel in Schritt (3) der Variante A erhaltenen Säureverbindung der Formel IV, wobei R₁ ein Wasserstoffatom, ein Chloratom, einen C₁-C₃-Alkylrest mit einer linearen oder verzweigten Kohlenwasserstoffkette oder einen 1-Oxo-C₁-C₅-alkylrest darstellt,
mit einer Verbindung der Formel VI: 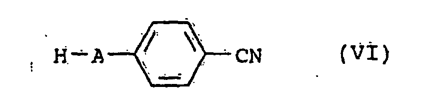 wobei A eine Gruppe 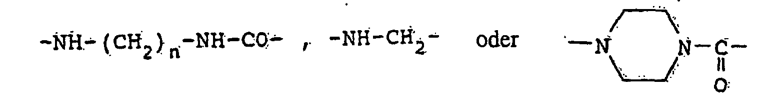 darstellt,
wobei n 2, 3 oder 4 ist,
unter Bedingungen, die denen des Schrittes (4) der vorstehend genannten Variante A analog sind, um eine Verbindung der Formel VII zu erhalten: 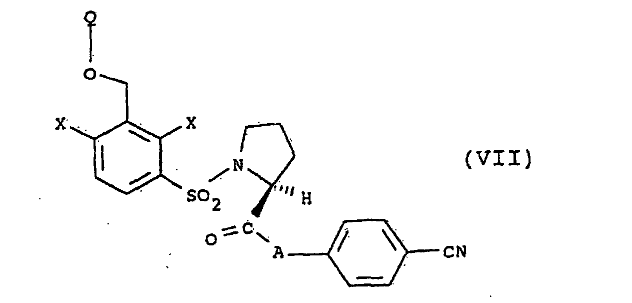 wobei Q, R₁, X und A dieselbe Bedeutung wie in den Ausgangsprodukten haben;
(2) Umsetzen der so erhaltenen Verbindung der Formel VII mit Hydroxylamin in einem Lösungsmittel bei Raumtemperatur (15-25°C) für 1 bis 12 Stunden, um eine Verbindung der Formel VIII zu erhalten: 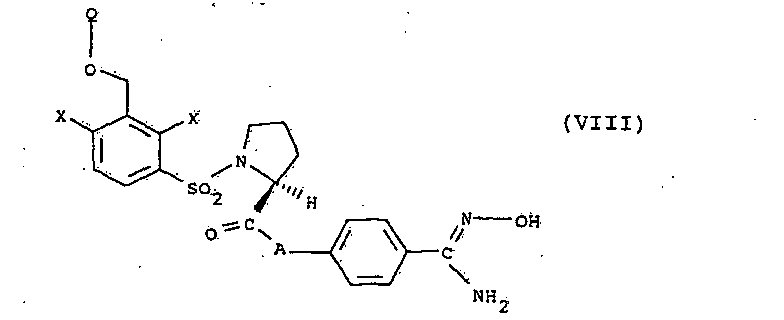 wobei Q, R₁, X und A die vorstehend genannte Bedeutung haben;
(3) Acetylieren der so erhaltenen Verbindung der Formel VIII bei einer Temperatur nahe der Raumtemperatur für 1 bis 8 Stunden, um die Verbindung der Formel IX zu erhalten: 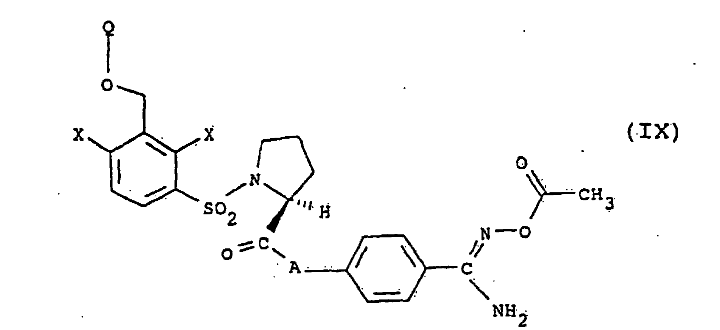 wobei Q, R₁, X und A die vorstehend angegebene Bedeutung haben;
(4) Durchführen einer Reduktion durch katalytische Hydrierung der so erhaltenen Verbindung der Formel IX bei einer Temperatur nahe der Raumtemperatur, unter einem Wasserstoffdruck zwischen 10⁵ und 10⁶ Pascal, um die Verbindung der Formel I zu erhalten, wobei Q, R₁, X und A die vorstehend angegebene Bedeutung haben und R₂ ein Wasserstoffatom ist;
dass es gemäß einer dritten Variante C die Schritte umfasst:
(1) Umsetzen der gemäß Schritt (3) der Variante A erhaltenen Verbindung der Formel V mit einem Amin der Formel 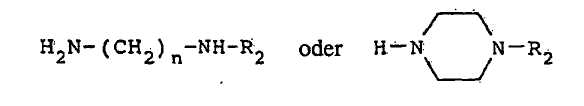 wobei n 2, 3 oder 4 ist und R₂ eine Aminoschutzgruppe, insbesondere die 1,1-Dimethylethoxycarbonylgruppe, darstellt,
unter Arbeitsbedingungen, die denen des Schrittes (4) der Variante A analog sind um eine Verbindung der Formel X zu erhalten: 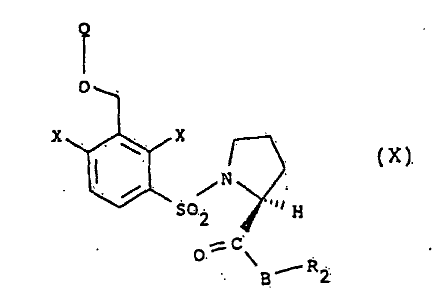 wobei B 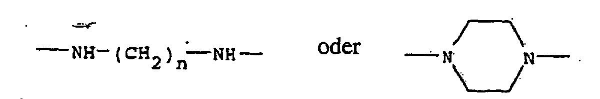 darstellt
und Q, R₁ und X die vorstehend angegebene Bedeutung haben;
(2) Durchführen der Entschützung der Aminfunktion der so erhaltenen Verbindung der Formel X durch Ersetzen des Restes R₂ durch ein Wasserstoffatom und Erhalten der Verbindung der Formel XI: 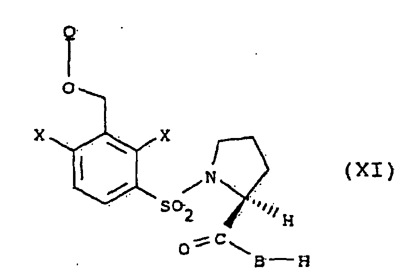 wobei B, Q, R₁ und X die vorstehend angegebene Bedeutung haben; und
(3) Umsetzen der so erhaltenen Verbindung der Formel XI mit 4-(Aminoiminomethyl)benzoesäure, unter Bedingungen, die denen des Schrittes (4) der vorstehend genannten Variante A analog sind um die Verbindung der Formel I zu erhalten, wobei R₂ ein Wasserstoffatom ist: 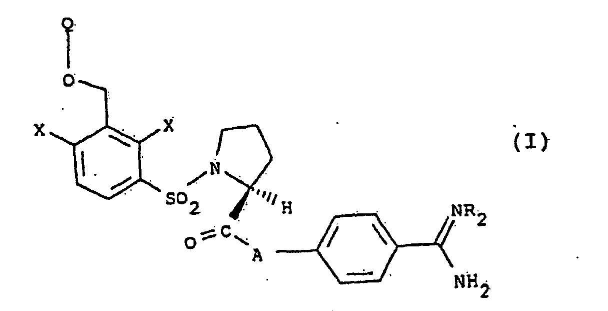 wobei
Q, R₁ und X die vorstehend genannte Bedeutung haben, R₂ ein Wasserstoffatom ist und A eine Gruppe: 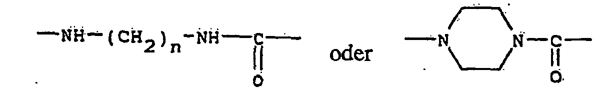 darstellt,
und dass es gemäß einer vierten Variante D die Schritte umfasst:
(1) Umsetzen der Säure der Formel: 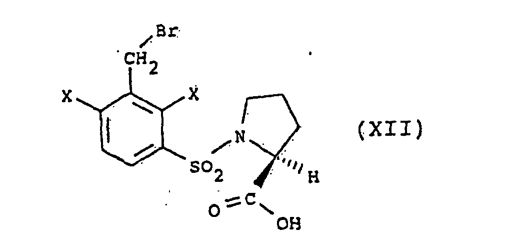 wobei X ein Halogenatom darstellt,
mit einer Verbindung der Formel: 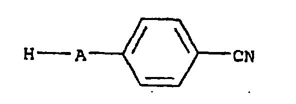 wobei A eine Gruppe: 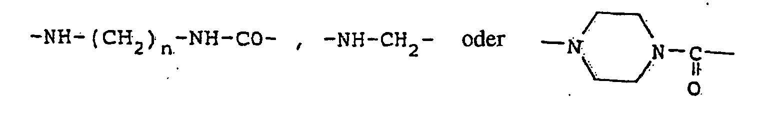 darstellt,
wobei n 2, 3 oder 4 ist,
unter Bedingungen, die denen des Schrittes (4) der vorstehend genannten Variante A analog sind und Erhalten der Verbindung der Formel XIII: 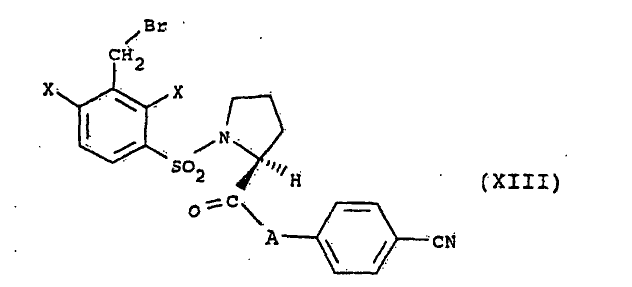 wobei A und X die vorstehend angegebene Bedeutung haben;
(2) Umsetzen der so erhaltenen Verbindung der Formel XIII mit einem hydroxylierten heterocyclischen Derivat der allgemeinen Struktur Q-OH, wobei Q ausgewählt ist aus den Strukturen: 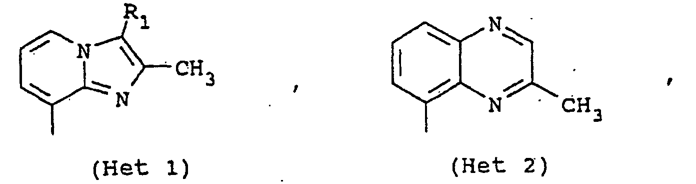 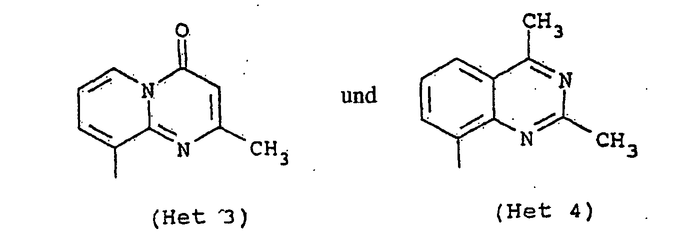 wobei R₁ ein Wasserstoffatom, ein Halogenatom, einen C₁-C₃-Alkylrest oder einen 1-Oxo-C₁-C₅-alkylrest darstellt,
unter Bedingungen, die denen des Schrittes (1) der vorstehend genannten Variante A analog sind um die Verbindung der Formel XIV zu erhalten: 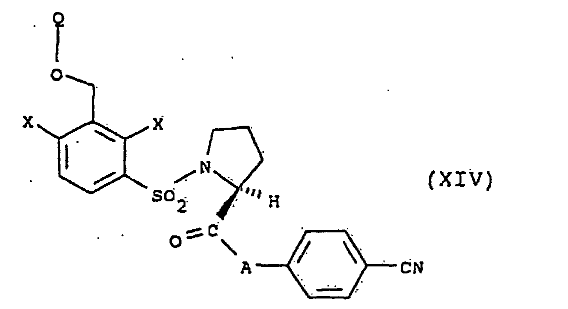 wobei Q, R₁, X und A dieselbe Bedeutung wie in den Ausgangsprodukten haben; und
(3) anschließend Durchführen einer Reihe von Reaktionen, die den zuvor in den Schritten (2), (3) und (4) der vorstehend genannten Variante B beschriebenen analog sind mit der Verbindung der Formel XIV, um die Verbindung der Formel I zu erhalten, wobei Q, X, R₁ und A dieselbe Bedeutung haben wie in den Ausgangsverbindungen.

4. Arzneimittel, **dadurch gekennzeichnet, dass** es, in Verbindung mit einem physiologisch verträglichen Exzipienten, mindestens eine Verbindung ausgewählt aus den Verbindungen der Formel I und ihren nicht-toxischen Additionssalzen gemäß Anspruch 1 umfasst.

5. Verwendung einer antagonistisch auf einen Rezeptor des Bradykinins und analoger Hormone wirkenden Substanz, wobei die Verwendung **dadurch gekennzeichnet ist, dass** man dabei auf eine antagonistisch auf den Bradykinin-B₂-Rezeptor wirkende Substanz, ausgewählt aus Verbindungen der Formel I und ihren nicht-toxischen Additionssalzen gemäß Anspruch 1, zurückgreift, zur Herstellung eines Medikaments zur Verwendung in der Therapie von pathologischen Zuständen, in die Bradykinin oder seine Homologen involviert sind.

6. Verwendung gemäß Anspruch 5 einer Substanz, ausgewählt aus den Verbindungen der Formel I und ihren nicht-toxischen Additionssalzen gemäß Anspruch 1, zur Herstellung eines Medikaments zum therapeutischen Einsatz bei der Behandlung schmerzhafter Zustände.

7. Verwendung gemäß Anspruch 5 einer Substanz, ausgewählt aus den Verbindungen der Formel I und ihren nicht-toxischen Additionssalzen gemäß Anspruch 1, zur Herstellung eines Medikaments zum therapeutischen Einsatz bei der Behandlung entzündlicher Zustände.

8. Verwendung einer Verbindung der Formel I oder eines ihrer Additionssalze gemäß Anspruch 1 als biologisches Reagens zur Bewertung der Affinität einer chemischen Verbindung zum Bradykinin-B₂-Rezeptor.

## Claims

1. N-(Benzenesulfonyl)-L-proline compound selected from the group consisting of:
(i) compounds of formula I: 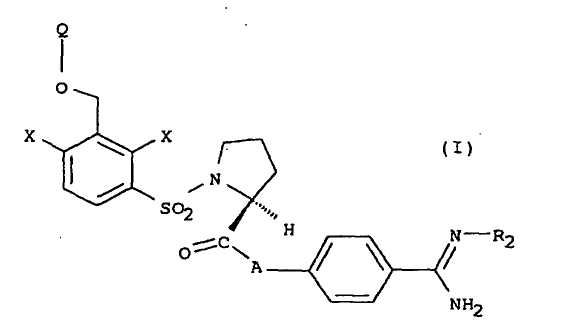 in which:
X is a halogen atom,
A is a divalent group 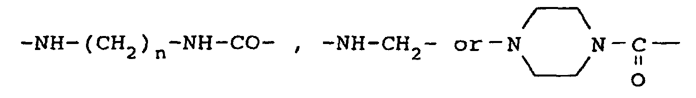
Q is a group 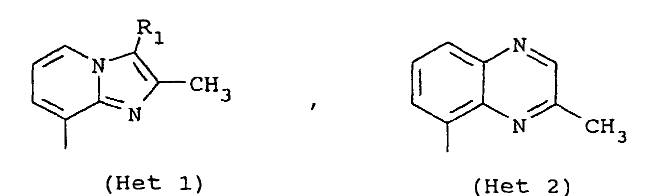 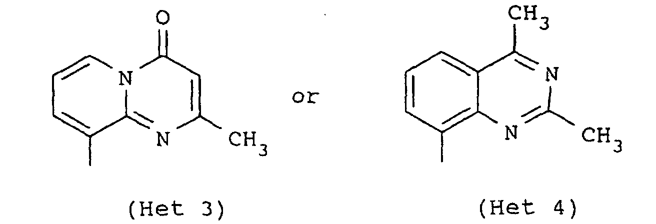
R₁ is a hydrogen atom, a halogen atom, a C₁-C₃ alkyl group with a linear or branched hydrocarbon chain, or a C₁-C₅ 1-oxoalkyl group,
R₂ is a hydrogen atom or an OH group, and
n is 2, 3 or 4; and
(ii) their addition salts.

2. Compound according to claim 1 wherein X is Cl.

3. Process for the preparation of a compound of formula I, which comprises, according to a first variant A, the steps which consist in:
(1) reacting a hydroxylated heterocyclic compound of the formula
Q-O-Met
in which:
Met is an alkali metal, especially Na or K, and
Q is a heterocyclic group selected from the structures Het 1, Het 2, Het 3 and Het 4: 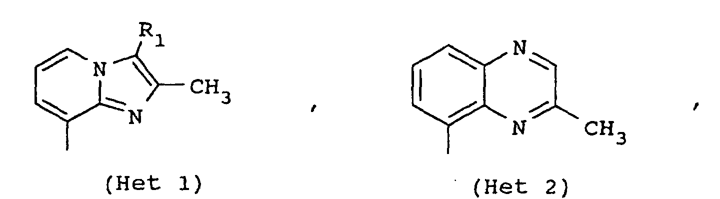 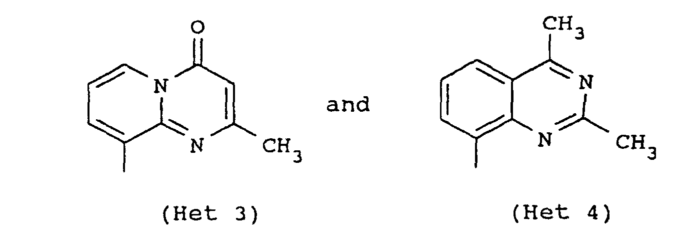
R₁ being a hydrogen atom or a C₁-C₃ alkyl group, with a compound of formula II: 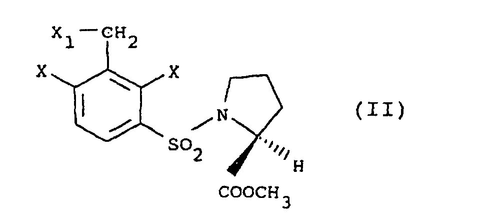 in which X is a halogen atom and X₁ is a halogen atom, preferably Br,
in an anhydrous solvent, at a temperature of between 0 and 50°C, for 0.5 to 10 hours, to give a compound of formula III: 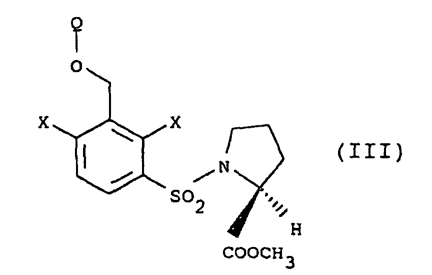 in which Q, X and R₁ are as defined above;
(2) if necessary, if Q in the compound of formula III is the group Het 1 in which R₁ is a hydrogen atom: 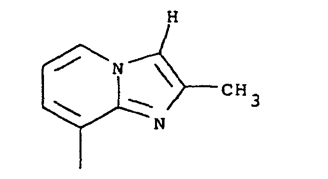 reacting said compound of formula III with a halogenating agent in a solvent, at a temperature of between about 0 and 50°C, for 0.5 to 20 hours, to give a compound of formula III': 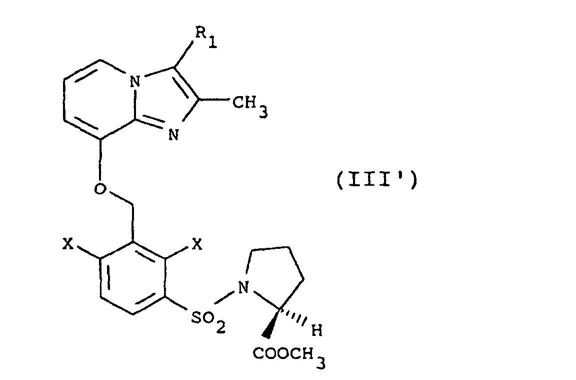 in which:
R₁ is a halogen atom, preferably Br or Cl;
(3) hydrolyzing the ester group of the compound of formula III or III' obtained according to one of steps (1) or (2) above, at a temperature of the order of 20 to 60°C, for 1 to 5 hours, to give a compound of formula IV: 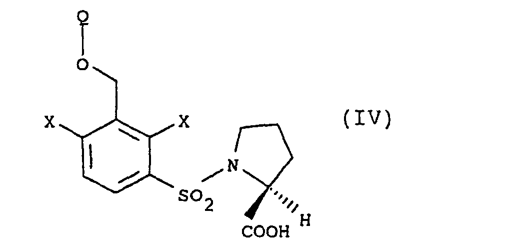 in which Q and X are as defined above and R₁ is a hydrogen atom, a halogen atom or a C₁-C₃ alkyl group;
(4) reacting the resulting compound of formula IV with the salt of an amine of the formula 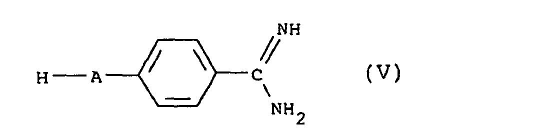 in which A is a group 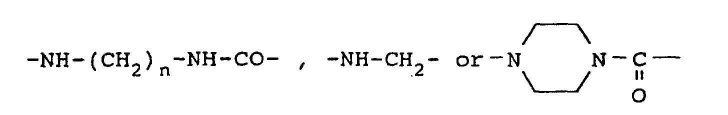 in which n is 2, 3 or 4,
in a solvent, in the presence of activators such as, in particular, 1-hydroxy-7-azabenzotriazole (HOAT) and 1-[3-(dimethylaminopropyl)-3-ethyl]carbodiimide (EDCI) hydrochloride, at a temperature close to room temperature (10 - 35°C), for 2 to 50 hours, to give a compound of formula I in which R₂ is H: 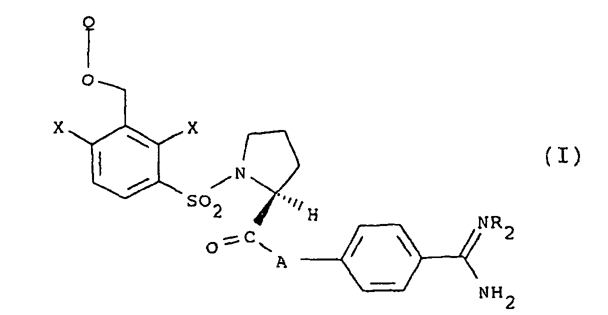 in which A, Q, X and R₁ are as defined above and R₂ is H; and
(5) if necessary, reacting the resulting compound of formula I with an acid to give the corresponding acid addition salt;
according to a second variant B, the steps which consist in:
(I) reacting the acid compound of formula IV, obtained for example in step (3) of variant A, in which R₁ is a hydrogen atom, a chlorine atom, a C₁-C₃ alkyl group with a linear or branched hydrocarbon chain, or a C₁-C₅ 1-oxoalkyl group, with a compound of formula VI: 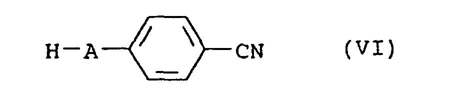 in which A is a group 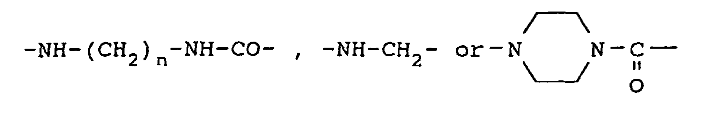 in which n is 2, 3 or 4,
under conditions analogous to those of step (4) of variant A above, to give a compound of formula VII: 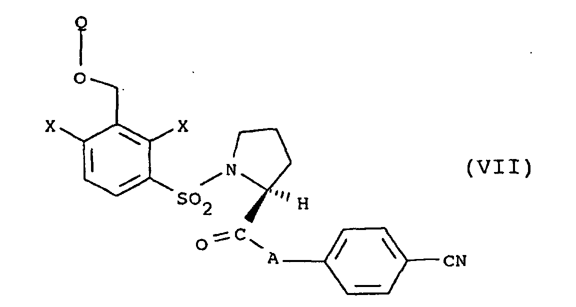 in which Q, R₁, X and A are as defined in the starting materials;
(2) reacting the resulting compound of formula VII with hydroxylamine in a solvent, at room temperature (15 - 25°C), for 1 to 12 hours, to give a compound of formula VIII: 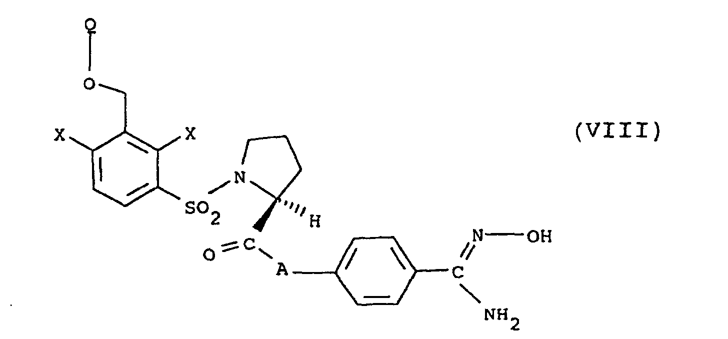 in which Q, R₁, X and A are as defined above;
(3) acetylating the resulting compound of formula VIII, at a temperature close to room temperature, for 1 to 8 hours, to give the compound of formula IX: 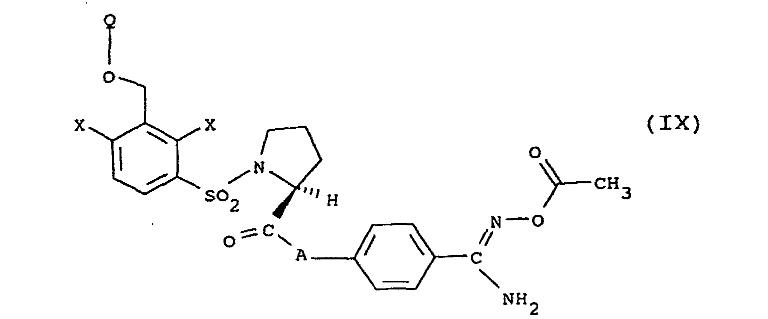 in which Q, R₁, X and A are as defined above; and
(4) reducing the resulting compound of formula IX by catalytic hydrogenation, at a temperature close to room temperature, under a hydrogen pressure of between 10⁵ and 10⁶ Pascals, to give the compound of formula I in which Q, R₁, X and A are as defined above and R₂ is H;
according to a third variant C, the steps which consist in:
(1) reacting the compound of formula V, obtained according to step (3) of variant A, with an amine of the formula 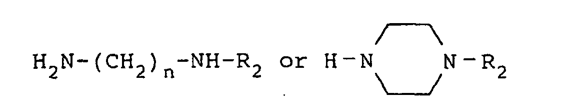 in which n is 2, 3 or 4 and R₂ is an amino-protecting group, especially the 1,1-dimethylethoxycarbonyl group,
under operating conditions analogous to those of step (4) of variant A, to give a compound of formula X: 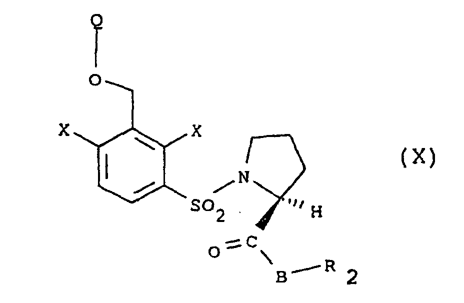 in which B is 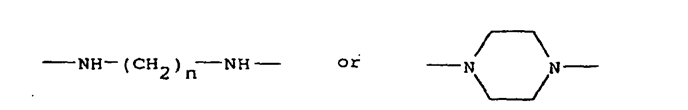 and Q, R₁ and X are as defined above;
(2) deprotecting the amine group of the resulting compound of formula X so as to replace the group R₂ with a hydrogen atom, to give a compound of formula XI: 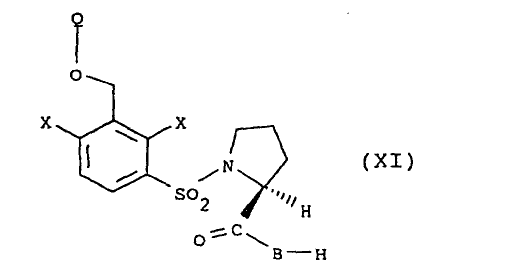 in which B, Q, R₁ and X are as defined above; and
(3) reacting the resulting compound of formula XI with 4-(aminoiminomethyl)-benzoic acid, under conditions analogous to those of step (4) of variant A above, to
give the compound of formula I in which R₂ is H: 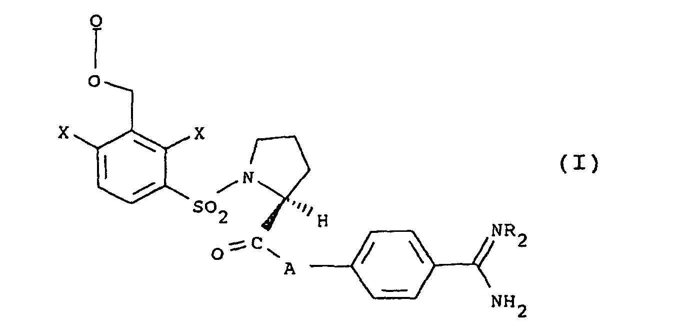 in which:
Q, R₁ and X are as defined above, R₂ is H and A is a group 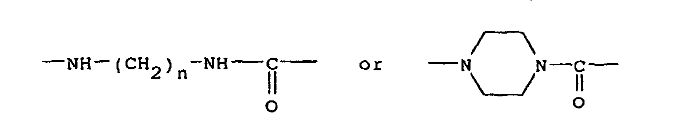 and according to a fourth variant D, the steps which consist in:
(1) reacting an acid of the formula 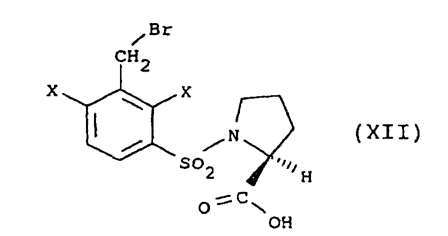 in which X is a halogen,
with a compound of the formula 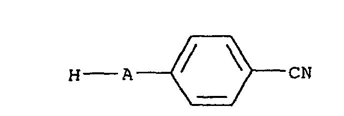 in which A is a group 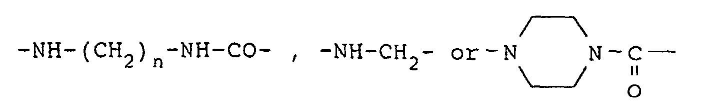 in which n is 2, 3 or 4,
under conditions analogous to those of (4) of variant A above, to give a compound of formula XIII: 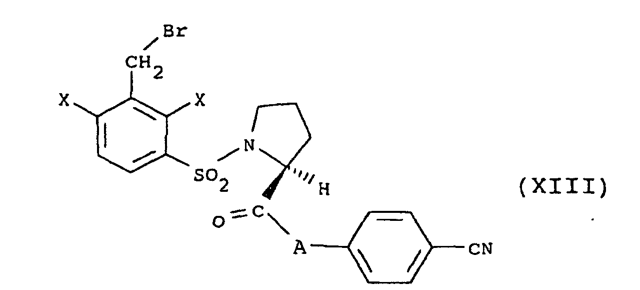 in which A and X are as defined above;
(2) reacting the resulting compound of formula XIII with a hydroxylated heterocyclic derivative of the general structure Q-OH, Q being selected from the structures 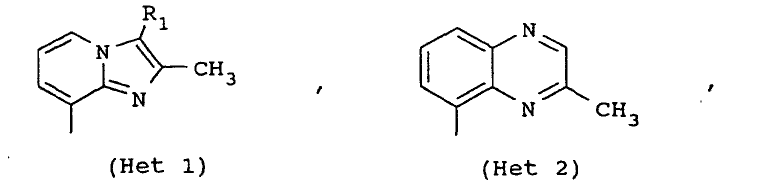 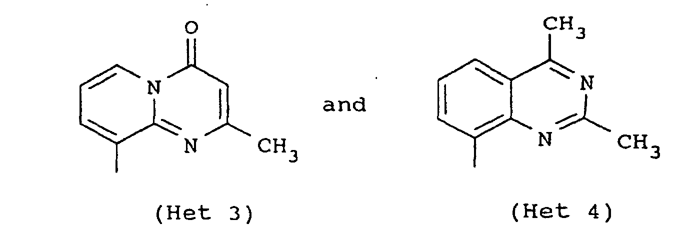 in which R₁ is a hydrogen atom, a halogen atom, a C₁-C₃ alkyl group or a C₁-C₅ 1-oxoalkyl group,
under conditions analogous to those of step (1) of variant A above, to give a compound of formula XIV: 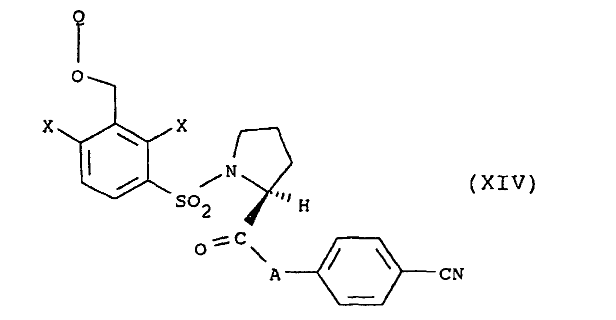 in which Q, R₁, X and A are as defined for the starting materials; and
(3) then performing, on the resulting compound of formula XIV, a series of reactions analogous to those previously described in steps (2), (3) and (4) of variant B above, to give the compound of formula I in which Q, X, R₁ and A are as defined for the starting compounds:

4. Therapeutic composition which contains, in association with a physiologically acceptable excipient, at least one compound selected from the group consisting of the compounds of formula I and their non-toxic addition salts according to claim 1.

5. Use of an antagonist of a receptor of bradykinin and analogous hormones, wherein a bradykinin B₂ receptor antagonist selected from the group consisting of the compounds of formula I and their non-toxic addition salts according to claim 1 is used to obtain a drug intended for use in therapeutics to combat pathological conditions involving bradykinin or its homologs.

6. Use according to claim 5 of a substance selected from the group consisting of the compounds of formula I and their non-toxic addition salts according to claim 1 for obtaining a drug intended for use in therapeutics for the treatment of painful states.

7. Use according to claim 5 of a substance selected from the group consisting of the compounds of formula I and their non-toxic addition salts according to claim 1 for obtaining a drug intended for use in therapeutics for the treatment of inflammatory states.

8. Use of a compound of formula I or one of its addition salts according to claim 1 as a biological reagent enabling the evaluation of the affinity of a chemical compound for the bradykinin B₂ receptor.
